(19)

Europäisches Patentamt
European Patent Office
Office européen des brevets

(11) **EP 2 949 746 A1**

(12) **EUROPEAN PATENT APPLICATION**
published in accordance with Art. 153(4) EPC

(43) Date of publication:
**02.12.2015 Bulletin 2015/49**

(21) Application number: **14743585.3**

(22) Date of filing: **23.01.2014**

(51) Int Cl.:
*C12N 5/07* (2010.01)    *C12M 1/18* (2006.01)
*C12N 5/071* (2010.01)

(86) International application number:
**PCT/JP2014/051362**

(87) International publication number:
**WO 2014/115799 (31.07.2014 Gazette 2014/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.01.2013 JP 2013010161**

(71) Applicant: **Tokyo Electron Limited**
**Tokyo 107-6325 (JP)**

(72) Inventors:
• **OZAKI, Shigenori**
**Minato-ku**
**Tokyo 107-6325 (JP)**
• **GOMI, Shinichi**
**Minato-ku**
**Tokyo 107-6325 (JP)**
• **KURAKAZU, Tomoaki**
**Stevenage, Herts SG1 2FX (GB)**
• **OSHIMA, Yasuhiro**
**Stevenage, Herts SG1 2FX (GB)**
• **KAWAMATA, Shin**
**Kobe-shi**
**Hyogo 650-0047 (JP)**
• **NISHISHITA, Naoki**
**Kobe-shi**
**Hyogo 650-0047 (JP)**

(74) Representative: **Diehl & Partner GbR**
**Patentanwälte**
**Erika-Mann-Strasse 9**
**80636 München (DE)**

(54) **METHOD OF SUBCULTURING PLURIPOTENT STEM CELLS**

(57)    The present invention provides a method of simply and uniformly subculturing pluripotent stem cells. The method includes the steps of dispersing cell masses obtained from passaging of the pluripotent stem cells into a single cell level, and subsequently, rapidly forming cell aggregates.

FIG. 5

EP 2 949 746 A1

## Description

CROSS REFERENCE TO RELATED APPLICATION

This application claims the priority benefit of Japanese Patent Application No. 2013-010161, filed January 23, 2013, which is hereby incorporated by reference in its entirety.

TECHNICAL FIELD

The present invention relates to a method of subculturing pluripotent stem cells. More particularly, the present invention relates to a method of conveniently and uniformly subculturing pluripotent stem cells.

BACKGROUND

In adherent cultures, cells are detached from the surface of the culture vessel by enzyme treatment or use of a cell scraper, and then passaged to a new culture vessel containing fresh medium. Pluripotent stem cells, when dissociated into single cells, are known to result in cell death, and thus must be passaged in a state of cell masses.. Thus, in the subculturing of pluripotent stem cells, when cells are detached from the culture vessel, pluripotent stem cells are detached in a state of colonies; broken down into cell masses with appropriate sizes by pipetting, etc.; and then seeded onto a new culture dish.

However, in these methods, there are problems in that the size of the cell mass varies depending on the pipetting technique and the sizes of the obtained cell masses are not uniform. If the sizes of the cell masses change by the pipetting work at the time of passaging, variations in the colony size may occur during culture following the passaging, which cannot be said to be perfect in terms of quality management for the cells. In addition, when variations in the sizes of the colonies occur, even though a certain colony has reached a particular size for passaging, cells in other colonies may not have sufficiently proliferated. For the above reason, variations in the size of the colonies in culture has a negative effect in terms of the cell yield efficiency.

Methods of dissociating pluripotent stem cells into single cells and seeding them have been known, but such methods are for cloning pluripotent stem cells having a single property and have a very poor efficiency in terms of cell proliferation. Thus, such methods are not suitable for the subculture of pluripotent stem cells (*see* Non-Patent Literature 1). Further, a method for efficiently inducing cell differentiation by aggregating pluripotent stem cells dissociated into single cells to form embryonic bodies having uniform sizes (*see* Non-Patent Literature 2), but such embryonic bodies are originally prepared to promote the induction of pluripotent stem cell differentiation (*see* Non-Patent Literatures 2 and 3). Once pluripotent stem cells initiate differentiation, they are considered to lose pluripotency, and thus there has been no attempt to form embryonic bodies of pluripotent stem cells for the maintenance of the undifferentiated state.

For maintaining and culturing pluripotent stem cells in a large scale, it is necessary to establish a method of efficiently and uniformly subculturing homogenized pluripotent stem cells by means as convenient as possible. However, a method for uniformly subculturing pluripotent stem cells while maintaining their undifferentiated state has not yet been established. Further, for maintaining and culturing pluripotent stem cells in a large scale, it is necessary to establish a method of subculturing pluripotent stem cells that is suitable for a total automation system, but no such methods are known.

[Prior Art Documents]

Non-Patent Literatures

Non-Patent Literature 1: Watanabe, K., et. al., "A ROCK inhibitor permits survival of dissociated human embryonic stem cells", Nature bBotechnology (2007) 25:681
Non-Patent Literature 2: Spelke D.P., et. al., "Methods for embryoid body formation: the microwell approach", Methods in Molecular Biology (2011) 690:151-162
Non-Patent Literature 3: Shimazaki Takuya, Okada Yohei, Yosijaki Dhakahito and Okano Hideyuki, "Protein, Nucleic acid and Enzyme", Kioritz Publication (2006) 51(13):1854-1861

SUMMARY

Accordingly, it is an object of the present invention to provide a method of efficiently and uniformly subculturing pluripotent stem cells.

The present inventors have found that when pluripotent stem cells are passaged, even in cases where cell masses obtained in culture were dispersed into single cells, cell death due to dispersion into single cells can be prevented by rapidly reforming cell masses (i.e., by forming cell aggregates) thereafter; and cell aggregates having uniform sizes and shapes can be obtained by employing a method of forming cell aggregates. Further, the present inventors have discovered that the cell aggregates thus obtained, although in which cells are aggregated in three dimensions, when seeded onto a dish, rapidly spread on the cell culture side of the dish and favorably and uniformly proliferate; and then form favorable undifferentiated colonies of pluripotent stem cells when continuously cultured. In addition, the cell aggregates thus obtained can be maintained and cultured in a favorable undifferentiated state during several passages. As a result of more detailed analyses, the present inventors have found that the efficiency of cell culture can be easily improved by controlling the size of the cell aggregates. Further, the present inventors have found that the ratio of undifferentiated cells among the cells being passaged can be elevated by sorting cells dispersed into a single cell level based on their sizes. Furthermore, the present inventors have learned that cell aggregates formed in the wells can be dropped onto the culture side of the culture vessel by inverting a vessel equipped with the wells,, which results in being able to seed (precisely seed) them onto certain positions in the culture vessel. The present invention is based on such knowledge as described above.

In accordance with one aspect of the present invention, the following methods are provided.

(1) A method of subculturing pluripotent stem cells, comprising the steps of:

(a) dispersing cell masses of pluripotent stem cells during passaging;
(b) seeding the dispersed cells in microwells;
(c) forming cell aggregates from the seeded cells in the microwells; and
(d) seeding the formed cell aggregates on a culture side of a culture vessel.

(2) The method of (1), wherein step (d) comprises the step of (d') inverting a vessel equipped with microwells to drop cell aggregates onto a culture side of a culture vessel.

(3) The method of (1) or (2), wherein in step (a), the cell masses are dissociated into cell masses each containing 1 to 100 cells.

(4) The method of (3), wherein in step (a), the cell masses are dissociated into cell masses each containing 1 to 10 cells.

(5) The method of (4), wherein in step (a), the cell masses are dissociated into single cells.

(6) The method of any one of (1) to (5), wherein in step (b), an average number of cells being seeded in each microwell is 10 to 3,500 cells per well.

(7) The method of (6), wherein in step (b), an average number of cells being seeded in each microwell is 25 to 870 cells per well.

(8) The method of (7), wherein in step (b), an average number of cells being seeded in each microwell is 40 to 500 cells per well.

(9) The method of (8), wherein in step (b), an average number of cells being seeded in each microwell is 55 to 220 cells per well.

(10) The method of any one of (1) to (9), wherein step (c) comprises the step of statically incubating the cells in the microwells for a sufficient time to form cell aggregates.

(11) The method of (10), wherein step (c) comprises the step of statically incubating the cells in the microwells for 8 to 24 hours.

(12) The method of (11), wherein the step (c) comprises the step of statically incubating the cells in the microwells for 8 to 12 hours.

(13) The method of any one of (1) to (12), wherein step (c) is carried out without the use of centrifugation.

(14) The method of any one of (1) to (13), wherein the pluripotent stem cells are human pluripotent stem cells.

(15) The method of (14), wherein the human pluripotent stem cells are human ES cells or human iPS cells.

(16) The method of any one of (1) to (15), which further comprises the step of (a') removing the differentiated cells after step (a).

(17) The method of (16), wherein the step of (a') removing the differentiated cells comprises the step of removing differentiated cells by sorting.

(18) The method of (17), wherein cells having a diameter more than the threshold value (the threshold value is more than or equal to 20 $\mu$m) are removed by sorting.

(19) The method of (18), wherein cells having a diameter more than the threshold value (the threshold value is more than or equal to 23 $\mu$m) are removed by sorting.

(20) The method of (16), wherein the step of (a') removing the differentiated cells is conducted based on whether a cell surface marker is expressed or not.

(21) The method of (20), wherein the cell surface marker is an undifferentiation marker expressed on the surface of the pluripotent stem cells.

(22) The method of (21), wherein the undifferentiation marker is one or more undifferentiation markers selected from the group consisting of alkali phosphatase, SSEA-3, SSEA-4, TRA-1-60 and TRA-1-81.

(23) A closed culture vessel in which a side equipped with microwells and a culture side are equipped and the both sides are placed opposite to each other.

(24) The closed culture vessel of (23), wherein the microwells each have a shape in which the inner circumference becomes smaller toward the bottom.

(25) The closed culture vessel of (24), wherein the microwells each have a rounded bottom, a V bottom, a U bottom or a chamfer plane bottom.

(26) The closed culture vessel of any one of (23) to (25), which is equipped with arrayed multiple microwells.

(27) The method of (2), wherein step (d') is conducted by inverting the closed culture vessel of any one of (23) to (26).

(28) A totally automated subculture system of pluripotent stem cells for practicing the method of any one of (1) to (22) and (27).

The method of the present invention is advantageous in that homogenous cell aggregates of pluripotent stem cells can be conveniently and rapidly obtained, which allows pluripotent stem cells to be stably passaged. Further, the method of the present invention is advantageous in that the seeded positions of cell aggregates can be controlled, for example, cells can be uniformly seeded, by inverting a culture vessel equipped with microwells to drop the cell aggregates onto a culture vessel. Furthermore, the method of the present invention is advantageous in that the ratio of undifferentiated cells can be elevated by removing differentiated cells during passaging. In addition, the method of the present invention of subculturing pluripotent stem cells is suitable for total automation, and all procedures in the present invention can be totally automated.

BRIEF DESCRIPTION OF THE DRAWINGS

FIG. 1 shows images showing the cell status of human iPS cells, which were dispersed into a single cell level and seeded in AggreWell 800, observed immediately and at 1 day after being seeded (**FIG. 1A**), and the cell aggregates thus obtained (**FIG. 1B**).

FIG. 2 shows phase contrast microphotographs for cells after cell aggregates obtained with or without centrifugation were passaged. The dark-colored parts in the cell aggregates or cell colonies are parts where cells are multilayered.

FIG. 3 is a graph showing the growth curve of cell aggregates obtained after passaging. The growth of cells was determined by measuring the area occupied by cells-($mm^2$) on the dish.

FIG. 4 shows immunofluorescence images of colonies after passaging.

FIG. 5 shows time-lapse microphotographs obtained by visually observing the process of the formation of cell aggregates on AggreWell.

FIG. 6 shows the static incubation times and phase contrast microphotographs of cells after incubation on AggreWell. In FIG.6, the phase contrast microphotographs were taken at 48 hours after the cells were seeded on AggreWell.

FIG. 7 shows the static incubation times and phase contrast microphotographs of cell after incubation on AggreWell. In FIG.7, the phase contrast microphotographs were taken at 168 hours after seeding the cells on AggreWell.

FIG. 8 shows phase contrast microphotographs showing the cell status at 7 days after being seeded when the conditions for forming cell aggregates were changed.

FIG. 9 shows phase contrast microphotographs showing the cell status at 7 days after being seeded when the conditions for forming cell aggregates were changed.

FIG. 10 shows phase contrast microphotographs showing the cell status at the 5th passage when cell aggregates were formed with centrifugation. In FIG.10, the spreading status and proliferation status of the cell aggregates at 2 to 7 days after being seeded are shown.

FIG. 11 is a graph showing the survival rates of colonies when the conditions for forming cell aggregates were changed. P1 to P5 means the passage numbers. Specifically, P1 represents data after the 1st passage, and P2 to P5 represents data after the 2nd to 5th passages, respectively. Data values exceeding 100% in the graph is considered to be due to the breakdown of the cell aggregates during passaging.

**FIG. 12** is a graph showing the ratio of multilayered colonies when the conditions for forming cell aggregates were changed.

**FIG. 13** is a graph showing the recovery rate of cells when the conditions for forming cell aggregates were changed. P1 to P5 means the passage numbers.

**FIG. 14** is a graph showing the death rate of cells when the conditions for forming cell aggregates were changed. P1 to P5 means the passage numbers.

**FIG. 15** is a graph showing the correlation between the number of cells contained in onecell aggregate and the diameter of the cell aggregate.

FIG. **16** is a graph showing the correlation between the number of cells contained in onecell aggregate and the spreading rate of cells after the cell aggregates were seeded.

FIG. **17** is a graph showing the correlation between the number of cells contained in onecell aggregate and the adhesion rate of cells after the cell aggregates were seeded.

FIG. **18** is a graph showing the correlation between the number of cells contained in onecell aggregate and the number of obtained cell aggregates when cell aggregates having uniform sizes were obtained by using 10,000 cells.

FIG. **19** is a graph showing the correlation between the number of cells contained in onecell aggregate and the proliferation rate of cells per one passage.

FIG. **20** is a graph showing the correlation between the number of cells contained in onecell aggregate and the number of days it took for the colony which was derived from each cell aggregate to reach the size of 2 mm in diameter.

FIG. **21** is a graph showing the correlation between the number of cells contained in onecell aggregate and the proliferation rate of cells per day.

FIG. **22** is a graph showing the correlation between the number of cells contained in onecell aggregate and the proliferation rate of cells after 45.42 days.

FIG. **23** is a graph showing the differences between the diameter of cells from favorable iPS colonies and the diameter of cells from poor iPS colonies.

FIG. **24** shows images showing the arrangements on the culture side of cell aggregates which were dropped onto the culture side of a culture vessel.

## DETAILED DESCRIPTION

The pluripotent stem cells used in the present invention may be pluripotent stem cells including embryonic stem cells (ES cells), induced pluripotent stem cells (iPS cells or artificial pluripotent stem cells), Muse cells (mutilinease-differentiating stress enduring cells), embryonic carcinoma cells (EC cells), embryonic germ cells (EG cells), etc., preferably EC cells or iPS cells. The pluripotent stem cells used in the present invention may also preferably be mammalian pluripotent stem cells including primate or rodent pluripotent stem cells, more preferably human pluripotent stem cells. The pluripotent stem cells used in the present invention may be most preferably human EC cells or iPS cells.

The method of the present invention of subculturing pluripotent stem cells may comprise the steps of

(a) dispersing cell masses of pluripotent stem cells during passaging;
(b) seeding the dispersed cells in microwells;
(c) forming cell aggregates from the seeded cells in the microwells with or without centrifugation; and
(d) seeding the formed cell aggregates on a culture side of a culture vessel.

The method of the present invention of subculturing pluripotent stem cells may be carried out in an adherent culture system. In the present invention, pluripotent stem cells can be maintained and cultured while maintaining favorable undifferentiated status.

The cell aggregates obtained in the method of the present invention of subculturing pluripotent stem cells may have loose adhesions between the cells. Thus, although cell death due to dispersion into single cells can be prevented, and the cell aggregates obtained in the method of the present invention can rapidly spread on the culture side of a culture vessel after passaging due to the loose adhesions between the cells. Also, the cell aggregates obtained in the method of the present invention, by inverting a vessel equipped with microwells to drop them onto the culture side of a culture vessel, may be seeded onto a controlled position in the culture vessel (hereinafter, often referred to as "precise seeding").

The following descriptions are intended to further illustrate each step of the method of the present invention of subculturing pluripotent stem cells.

(a) A process of dispersing cell masses of pluripotent stem cells during passage

(Detachment of cells from culture side)

In the present invention, in adherent cultures, pluripotent stem cells detached from the culture side through physiological or physical means can be used. In the present invention, as enzymes used for detaching pluripotent stem cells from the culture side, the conventional enzymes, for example, trypsin, dispase, accutase, collagenases, etc. may be used. Also, the detachment of pluripotent stem cells from the culture side may be conducted using a chemical substance having cell detaching activity including chelating agents for divalent cations ($Mg^{2+}$ in particular) such as ethylenediaminetetraacetic acid (EDTA), etc., which may be used together with the enzymes mentioned above. Further, in the present invention, in order to detach pluripotent stem cells from the culture side, vibration including high-frequency vibration may be applied to the culture side, and/or a cell scraper may be used. Furthermore, in the present invention, cells may be detached by a combination of the above physiological and physical detaching means. Those skilled in the art can detach pluripotent stem cells from the culture side by appropriately using the conventional methods as described above.

In the present invention, the cell masses may be dissociated into single cells. The dissociation of cell masses may be conducted, simultaneously with or after the process of detaching the cells from the culture side mentioned above.

(Dispersion of cells)

In the case where the cells detached from the culture side maintain the form of a cell mass, the detached cell masses may be dissociated into single cells by the liquid stream from the pipetting and then dispersed. In the present application, the term "dispersed into single cell levels" means that the cell masses are dissociated to cell masses each having an average number of contained cells per cell mass of 1 to 100 cells, preferably 1 to 10 cells and then dispersed, which may include cases where cell masses are dissociated completely into single cells and then dispersed. So, in the present invention, the cell masses may be completely dissociated into single cells and then dispersed; mostly dissociated into single cells and then dispersed; or mostly dissociated to cell masses each containing 1 to 100, preferably 1 to 10 cells and then dispersed. In case the dispersed cell masses are large, variation between the number of cells seeded in each microwell can easily occur when cell masses are seeded in the microwells, and thus it is preferable that the cell masses after the dispersing are small.

Further, the cell masses detached from the culture side may be further treated with an enzyme to be dispersed into a single cell level. The enzyme used for dissociating cells into a single cell level may be an enzyme capable of breaking adhesions between cell-cell or adhesions between cell-extracellular substrate (ECM), which are well known to those skilled in the art. The dissociation of cell masses using enzyme or liquid stream can be automated, and step (a) can be automated.

After the cells are dispersed by dissociating into a single cell level, chemical compounds suppressing the adverse effects (e.g., cell death, etc.) due to cell dispersion, for instance, ROCK inhibitors including Y-27632, etc., may be added to the suspension of dispersed cells.

(b) A process of seeding the dispersed cells in microwells

In accordance with the present invention, the cells or cell masses dispersed in step (a) (hereinafter, referred to as "cells") can proliferate well when passaged after forming cell aggregates therefrom. The formation of cell aggregates may be conducted by seeding the dispersed cells in microwells. The dispersed cells in step (a) naturally sink by gravity in the culture medium. Thus, if the cells are seeded in a well having a slope (deeply indented site), the cells are collected via the slope in the well. Then, the cells form cell adhesions with adjacent cells to form cell aggregates. Therefore, in the present invention, it is preferable that the well has a shape allowing cells to be collected by sinking, for example, a shape in which the inner circumference becomes smaller toward the bottom. In other words, the well may preferably have a shape that becomes narrower toward the bottom, for example, a shape of a horn, a rounded bottom, a V bottom, a U bottom or a chamfer plane bottom (a shape having a bottom without an angle and getting narrower toward the bottom). Further, the shape of an upper opening in each well may be appropriately selected considering the processability or multiple arrays of well, for instance, a shape of a polygon including a triangle, a quadrangle, a hexagon, etc., or a circle shape.

In the present invention, the prepared cell aggregates preferably have certain sizes and be even. In this regard, the sizes of cell aggregates may be determined depending on the number of cells seeded in each microwell, and therefore, in step (b), the number of cells seeded in each microwell may be a certain amount and even. In this regard, a certain amount means that the average number of cells seeded in each microwell in step (b), for example, may be 10 to 3,500 (i.e., the average diameter of the formed cell aggregates may be 35 to 350 $\mu$m), preferably 25 to 870 (i.e., the average diameter of the formed cell aggregates may be 50 to 200 $\mu$m), more preferably 40 to 500 (i.e., the average diameter of the formed cell aggregates may be 60 to 160 $\mu$m), most preferably 55 to 220 (i.e., the average diameter of the formed cell aggregates may be 69 to 115 $\mu$m). In order for the number of cells seeded in each microwell to be a certain amount evenly, the cell concentration in the cell suspension may be controlled, sufficiently suspended in the suspension, and then seeded.

In order to distinguish from the wells of the conventional cell culture plates, in the present application, the wells for forming cell aggregates are referred to as "microwells", and the term "microwells" is not intended to exclude wells having an upper opening of more than or equal to 1 mm in one side or diameter and includes a well having one side or an upper opening of more than or equal to 1 mm in diameter. The size of the upper opening of the microwell which may be determined depending on the sizes of the formed cell aggregates, for instance, may have areas the same as those of a circle of 100 $\mu$m to 3 mm in diameter, 200 $\mu$m to 800 $\mu$m in diameter, or 400 $\mu$m to 600 $\mu$m in diameter.

Further, in terms of obtaining cell aggregates in a large scale, it is preferable that the microwells be multiply arranged on the bottom side of a vessel and the microwells be also arranged without gaps (with no flat between adjacent wells) or with minimized gaps between wells. The arrangement of the wells on the bottom side of a vessel is illustrated in more detail in the following descriptions in step (d') regarding the process of precise seeding.

Furthermore, in terms of aligning the sizes of the formed cell aggregates, the shapes of microwells in a vessel may preferably be uniform. In this way, it is easy to relatively uniformly disperse cells in the microwells, which can result in the formation of cell aggregates having uniform sizes. Therefore, in the present invention, the dispersed cells in step (a) may be prepared by using a multiwell plate in which uniformly shaped microwells are multiply arranged on the bottom side. Such multiwell plates may include but are not limited to, for example, the commercially available AggreWell (trademark) (produced by STEMCELL Technologies Co.). Further, step (b) may be conducted using the closed culture vessel of the present invention as mentioned in the following descriptions, and may be conducted on the side equipped with microwells thereof.

In the present invention, the surfaces of microwells may be coated with a non-adhesive material.

The number of cells seeded in microwells may be appropriately controlled. Also, seeding of the cells in microwells can be uniformly conducted by sufficiently suspending the cells. Because such tasks can be automated, in step (b), the process of seeding a certain amount of cells in microwells can be automated.

<u>(c) A process of forming cell aggregates from cells seeded in microwells</u>

In accordance with the present invention, cells seeded in microwells may be statically incubated, to be collected on the bottom of the microwells by gravity, to adhere to adjacent cells, and to form cell aggregates. Cells may be collected on the bottom of the microwells by centrifuging using a centrifugal method.

In the case of using a centrifugal method, centrifugation may be conducted at 400 g to 3000 g for 1 to 10 min, but not be limited thereto. In this way, the cells can be effectively collected on the bottom of the microwells.

In accordance with the present invention, when cell aggregates are formed using a centrifugal method, it may be thought that the cells can be densely aggregated, but, in the present invention, the centrifugal method does not always need to be used. In other words, after the cells dispersed into a single cell level are seeded in the microwells, cell aggregates of pluripotent stem cells can be formed within several hours, without using a centrifugal method, for example, just by statically incubating the cells in the microwells. As such, in the present invention, cell aggregates can be formed without using a centrifugal method.

In the present invention, cell aggregates of pluripotent stem cells can be formed by statically incubating cells in the microwells. The static incubation time may be more than or equal to the time necessary for forming cell aggregates of pluripotent stem cells, for instance, more than or equal to 8 hrs. In terms of shortening the time for forming cell aggregates, the static incubation time may be 8 hrs to 24 hrs, preferably 8 to 16 hrs, more preferably 8 to 12 hrs. In the method of the present invention, as the time of static incubation becomes shorter, the adhesions between cells in the cell aggregates become looser, and thus cell aggregates can be easily broken up but can rapidly spread after being seeded in a vessel.

Cell aggregates prepared in step (c) of the present invention may preferably have certain sizes and be even. As described in step (b), the average number of cells contained in each cell aggregate may be, for instance, 10 to 3,500 cells (i.e., the average diameter of the formed cell aggregates may be 35 to 350 $\mu$m), preferably 25 to 870 cells (i.e., the average diameter of the formed cell aggregates may be 50 to 200 $\mu$m), more preferably 40 to 500 cells (i.e., the average diameter of the formed cell aggregates may be 60 to 160 $\mu$m), most preferably 55 to 220 cells (i.e., the average diameter of the formed cell aggregates may be 69 to 115 $\mu$m), and the average diameter of cell aggregates may be properly controlled depending on the sizes of the microwells and number of seeded cells. Further, microwells may be selected to have a size larger than the desired sizes of the prepared cell aggregates.

For example, in the case of preparing cell aggregates having an average diameter of 50 $\mu$m using human iPS cells, cells of $2.8 \times 10^4$ cells/well may be seeded in a 24-well plate (2 cm$^2$/well) equipped with 1200 microwells per well (where e.g., the microwell size is 400 $\mu$m $\times$ 400 $\mu$m). Also, for instance, in the case of preparing cell aggregates having an average diameter of 100 $\mu$m, cells of $1.8 \times 10^5$ cells/well may be seeded in a 24-well plate (2 cm$^2$/well) equipped with 1200 microwells. Further, for example, in the case of preparing cell aggregates having an average diameter of 200 $\mu$m, cells of $2.9 \times 10^5$ cells/well may be seeded in a 24-well plate (2 cm$^2$/well) equipped with 300 microwells per well (where e.g., the microwell size is 800 $\mu$m $\times$ 800 $\mu$m). Accordingly, about 23, about 151 and about 981 cells are contained in cell aggregates of human iPS cells having diameters of 50 $\mu$m, 100 $\mu$m and 200 $\mu$m, respectively. Those skilled in the art can obtain cell aggregates having the desired sizes by calculating the necessary number of cells depending on the diameters of the prepared cell aggregates.

Cells seeded in microwells can form cell aggregates by centrifugation or simple static incubation without centrifugation. Therefore, step (c) can be totally automated.

(d) A process of seeding the formed cell aggregates on the culture side of a culture vessel

Cell aggregates formed in a culture vessel equipped with microwells in step (c) may be seeded in a culture vessel (i.e., the culture side of a culture vessel) thereafter. Such a process of step (d) may be conducted by harvesting cell aggregates, suspending them in a medium, and seeding them on the culture side of a culture vessel, and step (d) can be totally automated. Seeded cell aggregates can rapidly spread after being seeded and then favorably cultured in a state maintaining the pluripotent property. In terms of uniformly seeding cell aggregates on the culture side of a culture vessel, a cell suspension containing cell aggregates may preferably be sufficiently suspended and then seeded.

In the method of the present invention, in step (d), the precise seeding of cell aggregates is possible. Specifically, in step (d), cell aggregates can be precisely seeded on the culture side of a culture vessel by conducting the step of (d') inverting a culture vessel equipped with microwells to drop cell aggregates onto the culture side of a culture vessel. In step (d'), if a culture vessel equipped with microwells is inverted, cell aggregates can be almost vertically dropped from the microwells onto the culture side of the culture vessel and thus can be seeded in an arrangement which is a mirror image of the arrangement of the inverted microwells. In this regard, the arrangement of the microwells may be designed based on the desired arrangement of dropping the cell aggregates (the same applies for the arrangement design of microwells in the vessel equipped with microwells used in step (b)), to seed cell aggregates at controlled positions (precise seeding). As such, in the method of the present invention, by conducting step (d') in step (d), cell aggregates can be precisely seeded on the culture side of a culture vessel. Precise seeding, for example, may be employed for uniformly seeding cell aggregates on the culture side of a culture vessel, and accordingly, cells can uniformly proliferate on the culture side.

In terms of efficiently using the culture side of a culture vessel, it is preferable that microwells, for instance, are arranged in the form of a honeycomb (the same for the arrangement design of microwells in the vessel equipped with microwells used in step (b)). As such, useless gaps between colonies formed from pluripotent stem cells can be minimized, and thus the culture side can be efficiently utilized. In the present application, microwells are arranged in the form of a honeycomb, which means that multiple microwell rows extended to one fixed direction may be formed; each microwell row comprises multiple microwells continuously arranged in the above one direction; and a microwell included in any one microwell row is arranged such that it alternates with microwells included in the adjacent microwell row. As described above, in the method of the present invention, step (d) may further comprise the step of (d') inverting a culture vessel equipped with microwells to drop cell aggregates onto the culture side of a culture vessel. Because the sinking speed of cell aggregates by gravity is not so fast, even in the case where cell aggregates are not adhered to the microwells, cell aggregates can be aligned relatively favorably on the culture side of a culture vessel by inverting the culture vessel equipped with microwells. In the case cell aggregates are adhered to the microwells, the adhesion between the microwells and the cell aggregates may be dissociated using shock, stream, vibration (e.g., low frequency vibration or high frequency vibration), etc.

In step (d'), inverting a culture vessel can be easily conducted using a closed culture vessel, for example, a closed culture vessel in which a side equipped with microwells and a culture side are equipped and both sides are placed opposite to each other.

Therefore, in the present invention, a closed culture vessel is provided in which a side equipped with microwells (preferably, arrayed multiple microwells) and a culture side, and both sides are placed opposite to each other. When the closed culture vessel is used, the seeding of dispersed cells in step (a) in microwells (corresponding to step (b)), for example, may be conducted by injecting a sufficiently suspended cell suspension in a vessel and then statically incubating the vessel, facing the side equipped with microwells down. Also, inverting the vessel thereafter may be carried out by inverting the whole vessel.

In accordance with the present invention, step (d') can be totally automated. In terms of facilitating the total automation of processes as mentioned above, it may be preferable to use the closed culture vessel of the present invention in step (d').

As described above, the method of the present invention of subculturing pluripotent stem cells may be carried out by conducting steps (a) to (d). As mentioned above, all these steps can be automated.

In accordance with the present invention, the method of the present invention of subculturing pluripotent stem cells may further comprise the step of (a') removing differentiated cells between steps (a) and (b).

(a') A process of removing differentiated cells

According to the method of the present invention, even in the case of dispersing pluripotent stem cells into single cells, adverse events including cell death can be suppressed. Further, by forming cell aggregates thereafter, cells can be efficiently passaged. In the present invention, one of the advantages of pluripotent stem cells being dispersed into single cells is that the isolation and removal of differentiated cells can be conducted based on the characteristic state at a single cell level. Step (a') is a process for isolation and removal of differentiated cells, which became applicable to the subculturing of pluripotent stem cells for the first time in the present invention where pluripotent stem cells can be dispersed into single cells. Namely, step (a') is a process based on the assumption that pluripotent stem cells are dispersed into single cells in step (a).

The present inventors, as described in the following Examples, have clarified that cells dispersed into single cells can be sorted into undifferentiated cells and cells which initiated differentiation based on their sizes. Specifically, in human iPS cells, undifferentiated cells have a diameter ranging from 14 to 20 $\mu$m centering around 17 $\mu$m in size but cells which initiate differentiation have a diameter ranging more than 23 $\mu$m in size. Based on these results, it is possible to passage cells with elevating ratio of undifferentiated cells by conducting the step of (a') sorting cells according to their size after step (a) to remove cells which initiated differentiation.

In accordance with the present invention, in step (a'), cells having a diameter exceeding the threshold value (the threshold value is more than or equal to 20 $\mu$m, preferably 23 $\mu$m) may be removed by sorting to elevate the ratio of undifferentiated cells. The threshold value for sorting in step (a') may be preferably less than or equal to 25 $\mu$m, for example, 20 $\mu$m, 21 $\mu$m, 22 $\mu$m, 23 $\mu$m, 24 $\mu$m or 25 $\mu$m, more preferably 23 $\mu$m, 24 $\mu$m or 25 $\mu$m, still more preferably 23 $\mu$m. If the threshold value is set lower, the ratio of differentiated cells mixed therein can decrease but simultaneously, the recovery rate of the undifferentiated cells also declines. Also, if the threshold value is set higher, though the recovery rate of undifferentiated cells can be enhanced, the ratio of differentiated cells mixed therein may also increase. A person skilled in the art can appropriately set a threshold value based on the recovery rate and mixed ratio of cells.

In the present invention, sorting cells in step (a') may be carried out by, but is not limited to, for example, using a cell isolation filter or a cell sorter. Cell isolation filters have been employed for isolating cells in suspension cell systems, where cells of variable sizes can be sorted. Cell isolation filters may include, for example, the commercially available Falcon S, size 20 $\mu$m (produced by Azone co., Serial no. 2-7210-01), etc. which can be used in the present invention. Besides, several methods for preparing cell isolation filters (e.g., Japanese Patent Publication No. 2002-178, etc.) are known, and therefore, those skilled in the art can prepare a cell isolation filter to sort cells. Further, cells may also be sorted using a cell sorter, and those skilled in the art can sort cells according to, for instance, the supplier's instruction manual, etc.

Further, the removal of differentiated cells may be conducted based on whether a marker expressed on the surface of the cells (a cell surface marker) is expressed or not. The cell surface marker which can be used in the removal of differentiated cells may include undifferentiation markers expressed in pluripotent stem cells, and the known undifferentiation markers may include, for example, alkali phosphatase, SSEA-3, SSEA-4, TRA-1-60, and TRA-1-81, etc. Several methods for isolating cells based on whether a cell surface marker is expressed or not have been well known, and a person skilled in the art can isolate and remove differentiated cells as appropriate. Isolation based on whether a cell surface marker is expressed or not may be conducted by employing technology such as flow cytometry, etc.

The isolation of differentiated cells or flow cytometry can be automated. Accordingly, step (a') can be automated.

As illustrated above, in the present invention, it is possible to automate all procedures of step (a), step (a'), step (b), step (c), step (d) and step (d'). Therefore, the method of the present invention can be totally automated.

Therefore, in accordance with the present invention, there is provided a totally automated subculture system of pluripotent stem cells for practicing the method of the present invention. The totally automated subculture system of pluripotent stem cells for practicing the method of the present invention may be equipped with one or more means selected from the group consisting of (1) means for culturing pluripotent stem cells; (2) means for detaching pluripotent stem cells from the culture sides and dispersing them into a single cell level; (3) means for seeding the dispersed cells on multimicrowell plates (wherein the multimicrowell plates may be centrifuged after seeding); and (4) means for seeding the formed cell aggregates on the culture sides of a culture vessel, preferably all of these means. In one embodiment of the present invention, the totally automated subculture system of pluripotent stem cells for practicing the method of the present invention may be equipped with the closed culture vessel of the present invention and the means of (4) may be accomplished by the means of (5) for inverting the closed culture vessel of the present invention.

Also, the resulting cells subcultured according to the method of the present invention may be frozen and preserved according to the conventional freeze-preservation method protocols. The frozen cells may be thawed and subjected to the procedure of step (a) and the following procedures thereof (e.g., the procedures of step (a') to step (d)).

In accordance with the present invention, after step (c), the resulting cell aggregates may be frozen and preserved. The freeze-preservation of cell aggregates may be conducted according to the conventional freeze-preservation method protocols, after centrifuging the cell aggregates to obtain a pellet. The frozen cells may be thawed and subjected to the procedure of step (a) and the following procedures thereof or the procedure of step (c) and the following procedures thereof (e.g., step (d)).

Freezing of the dispersed cells or cell aggregates may be carried out using the conventional cell freezing methods, and those skilled in the art can choose a freezing method as appropriate.

<Examples>

Example 1: Study of preparation of a single cell suspension of iPS cells and the subculture method thereafter

In the case pluripotent stem cells such as ES cells or iPS cells are scattered into single cells during passaging, cell death is induced. Also, there are concerns that the form of embryonic bodies allows cells to initiate differentiation. In this embodiment of the present invention, the cells were scattered into single cells and cell aggregates were allowed to be rapidly formed and passaged, and then it was examined whether such problems of cell death or cell differentiation would occurr.

In this embodiment, human iPS cells (cell lines established by the public foundation corporation high tech medical improvement foundation cell analysis group Kawamata laboratory) were used as cells. The cells were cultured under feederless conditions. The employed culture medium was ReproFF2 medium (produced by ReproCell co., serial no. RCHEMD006) supplemented with bFGF (produced by Waco jyunyakku Industry co., serial no. 064-04541) in a final concentration of 5 ng/ml. Also, 10-mm cell culture dishes (produced by BD co., serial no. REF353003) were used as culture vessels, and their insides were coated with ECM to assure the adhesive property of iPS cells to the culture dishes before being used in cell culture, according to the supplier's instruction manual. For the ECM, BD matrigel (produced by BD co., serial no. 356234) was used.

The cells were cultured until they became confluent according to the conventional method. Then, the used medium was removed by suction, and the cells were washed once with 10 ml of phosphate buffered saline (Life Technologies Co., serial no. 14190). Afterwards, the cells were treated with 1 ml of the Accutase solution (produced by Innovative Cell Technologies Co., serial no. AT104) at 37 degrees C for 5 min and harvested into a tube.

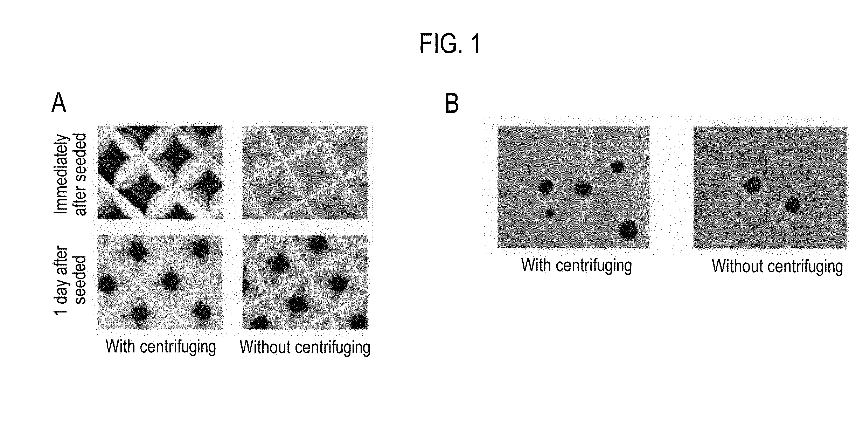

The cells thus obtained were centrifuged (440 g, 5 min) to be harvested, followed by removal of the supernatant through suction and resuspending the resulting cells with 1 ml of medium, and then ROCK inhibitor Y-27632 (produced by STEMGENT Co., serial no. 04-0012) was added thereto in a final concentration of 10 $\mu$M to obtain a cell suspension. Then, the resulting cell masses were broken down until single cells were obtained by the liquid flow of pipetting. Afterwards, the cell concentration of the cell suspension thus obtained was adjusted and the resulting cell suspension was placed in the wells of AggreWell 800 (produced by STEMCELL Technologies Co.). The AggreWell 800 was centrifuged (2,000 g, 5 min) or not, and cultured at 37 degrees C overnight to form cell aggregates. The state of the cells immediately after and 1 day after being seeded in cases where centrifugation was carried out or not are shown in **FIG. 1A.** As shown in **FIG. 1A,** in the case of centrifuging, the cells were collected on the bottom of the well having the shape of an inverted pyramid (**FIG. 1A -** upper left) from immediately after being seeded, but in case of no centrifuging, cells were not collected (**FIG. 1A -** upper right). However, at 1 day after being seeded, in both cases of centrifuging and no centrifuging, the cells were collected on the bottom to form cell aggregates. Also, the resulting cell aggregates had almost equal sizes (**FIG. 1B**).

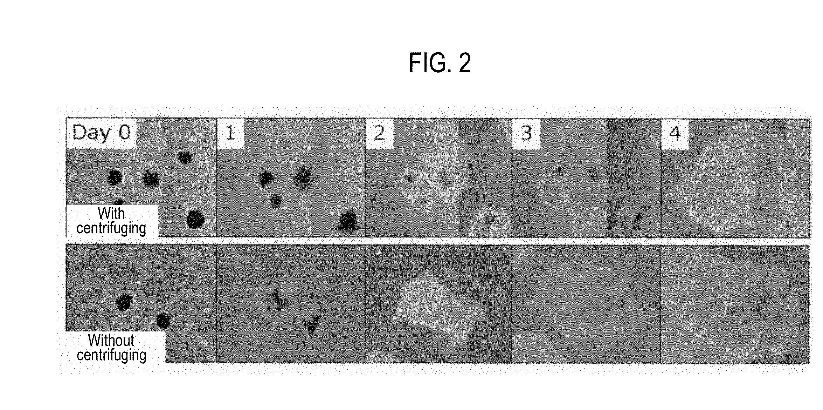

The obtained cell aggregates were harvested by pipetting without breaking them up as much as possible, and seeded in 6-well plates (300 cell aggregates/well). As a result of observing the cell growth using a phase contrast microscope after seeding, the cell aggregates favorably spread and then proliferated both cases of centrifuging or no centrifuging (FIG. 2).

The cell aggregates obtained in this embodiment of the present invention are very different from those obtained in the conventional passaging with regard to having a three-dimensional structure artificially aggregated, and in this embodiment, although the cell aggregates were prepared as described above, they naturally spread and formed cell colonies of pluripotent stem cells like those in the conventional culture in their culture, to be appropriately cultured.

In the case of not conducting centrifugation in the formation of cell aggregates, the resulting cell aggregates spread more rapidly than those in the case of centrifuging (**FIG. 2**, at 2 days after seeding). As shown in these results, with regard to the subculturing of iPS cells, more preferable results can be obtained in the case of no centrifugation.

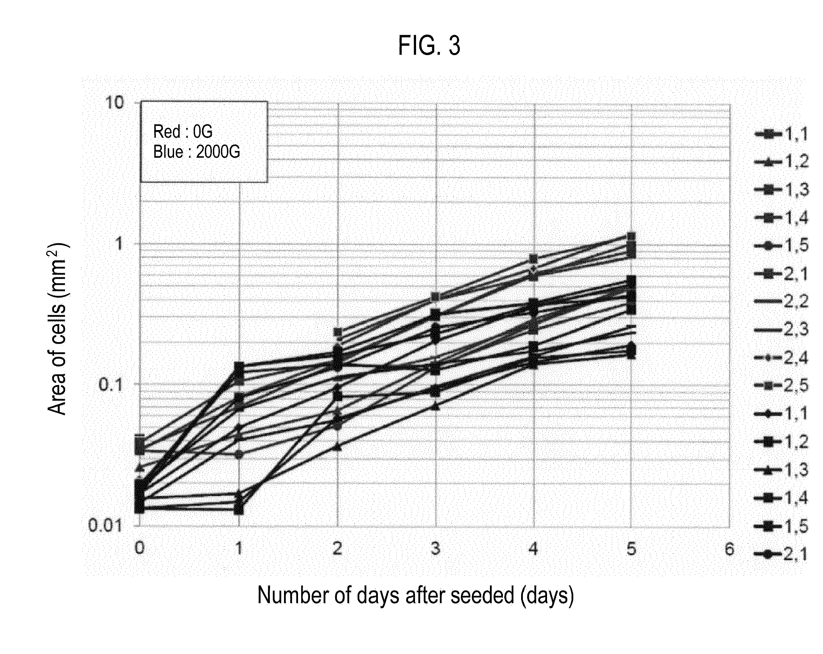

For a more detailed analysis, those in both cases were also compared by the growth curves of cells after being seeded in 6-well plates, but there were no significant differences between those in both cases (**FIG. 3**).

Thereafter, in order to verify that the cell aggregates are maintaining their undifferentiated status when continuously being cultured, the obtained cell aggregates were seeded in a 6-well plate, harvested after 5 days, fixed with formaldehyde, and subjected to an immunofluoresence staining analysis. Nuclear staining was carried out by incubating the cells in phosphate buffer saline containing 1 $\mu$g/ml of DAPI for 15 min. Immunofluoresence staining may be conducted using the anti-Nanog antibody (produced by ReproCell Co., serial no. RCAB0003P) for the analysis of Nanog expression and the anti-Oct3/4 antibody (produced by Santa Cruz Biotechnology Co., serial no. sc-5279) for Oct3/4 according to the conventional methods. Bright field images were taken by employing a phase contrast microscope (produced by Olympus Co., serial no. IX-81).

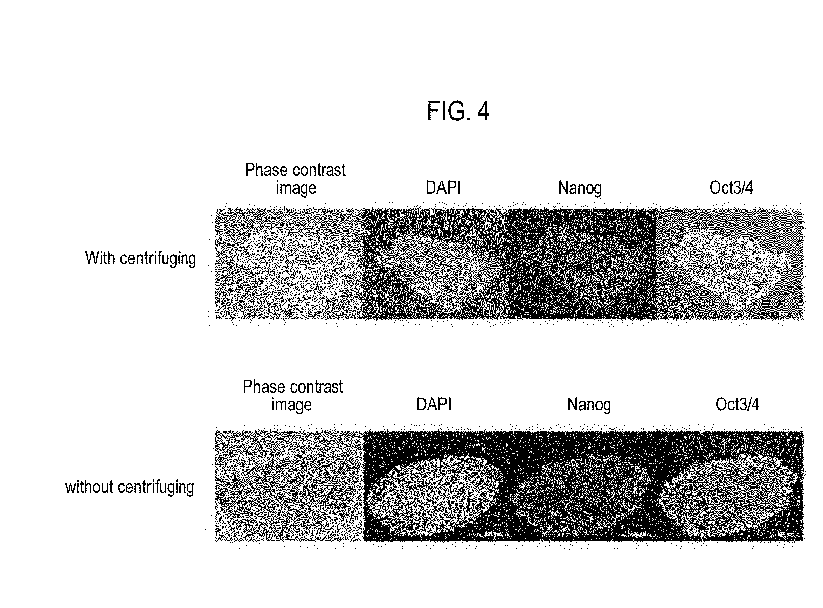

As a result, it was clear that all undifferentiation markers were expressed (**FIG. 4**).

These results suggest that even though the cells are scattered into the single cell level, cell death does not occurr by forming cell aggregates thereafter; the cell aggregates are rapidly (∼ 1 day) formed; and favorable colonies of pluripotent stem cells can be obtained from the formed aggregates and also cultured in their undifferentiated state.

Example 2: Study of the time of cell aggregate formation

In Example 1, the cell aggregates were formed using AggreWell, wherein the forming time was 24 hrs. In this embodiment of the present invention, the optimum time for forming cell aggregates was examined.

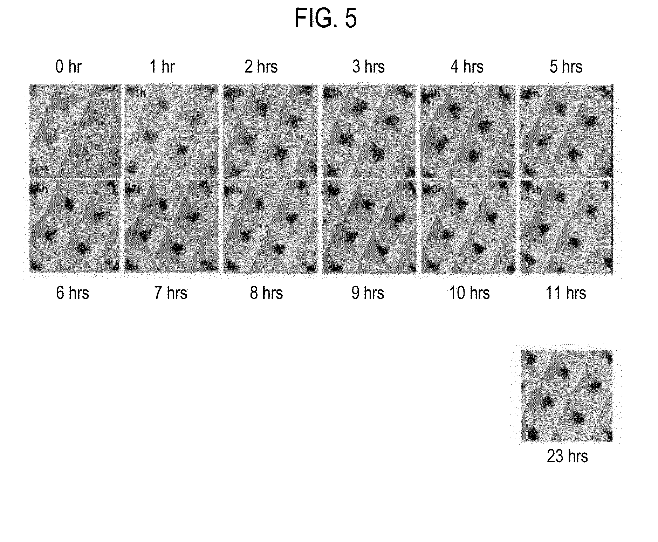

First, a cell suspension was obtained according to the same method as described in Example 1. The obtained cell suspension was placed in the wells of AggreWell, and then the formation of cell aggregates was monitored in a time-lapse manner (**FIG. 5**). Consequently, in the case of either centrifuging or not, no mass-shaped change of cells could be observed at 10 hrs. Perhaps, the reason for no change being observed is considered to be due to the formation of adhesions between cells.

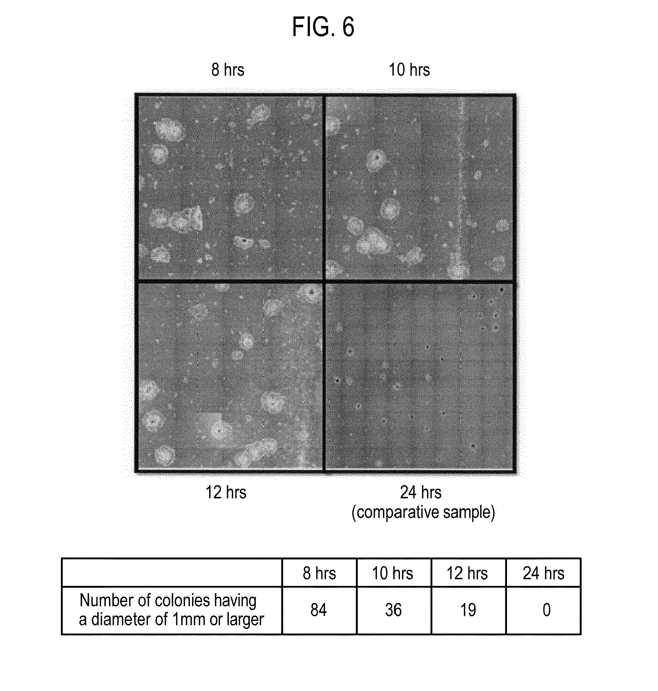

Accordingly, the obtained cell suspension was placed into the wells of AggreWell, statically incubated for 8 hrs, 10 hrs, 12 hrs and 24 hrs, respectively, and the cell aggregates harvested from the wells and seeded to 6-well plates without changing the cell masses. At 48 hrs after being placed, the number of colonies having a diameter of 1 mm or larger was counted. As a result, the number of colonies became higher when the static incubation time was shorter (**FIG. 6**). In this regard, these results indicate that the cell aggregates can spread faster into colonies by shortening the static incubation time.

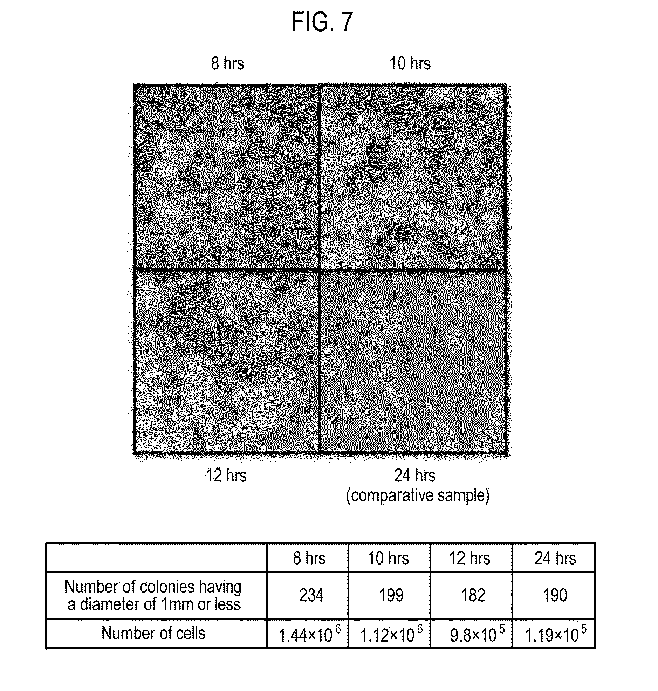

Then, the obtained cell suspension was placed into the wells of AggreWell, and observation for the appearances of the seeded cells was repeated at 168 hrs (7 days) after being placed, and all the cells favorably spread and proliferated. Therefore, as a result of counting the number of colonies smaller than 1 mm in diameter, it was clarified that the number of colonies having a diameter smaller than 1 mm when the static incubation time was 8 hrs was higher compared to those when the static incubation time was more than or equal to 10 hrs (**FIG. 7**). These results are considered to be due to the fact that as the static incubation time gets shorter, cell adhesions in cell aggregates become weaker, and the cell aggregates are easily broken down when being seeded in 6-well plates.

Further, the obtained cell suspension was placed into the wells of AggreWell, and the cell numbers were counted respectively at 168 hrs (7 days) after being placed. As a result, the cell number was significantly high when the static incubation time was 8 hrs but there were no such changes in those when the static incubation time was more than or equal to 10 hrs (**FIG. 7**).

These results demonstrate that the static incubation time in the wells of AggreWell of 8 hrs to 12 hrs is satisfactory.

Example 3-1: Study of the optimum size of cell aggregates

In this embodiment of the present invention, the optimum size of cell aggregates in the subculture thereof was examined.

First, a cell suspension was obtained according to the same method as described in Example 1. The obtained cell suspension was placed in the wells of AggreWell in order to form cell aggregates, with conditions for forming the cell aggregates set as described in Table 1.

<Table 1> Conditions for forming cell aggregates

| | Condition 1 | Condition 2 | Condition 3 | Condition 4 | Condition 5 | Condition 6 |
|---|---|---|---|---|---|---|
| Centrifugation | With centrifugation | | | Without centrifugation | | |
| Size of cell aggregates ($\mu$m) | 50 | 100 | 200 | 50 | 100 | 200 |
| Cell number | $2.8\times10^4$ | $1.8\times10^5$ | $2.9\times10^5$ | $2.8\times10^4$ | $1.8\times10^5$ | $2.9\times10^5$ |
| Number of cell aggregates [#1] | 1,200 | | 300 | 1,200 | | 300 |
| Type of AggreWell [#2] | 400 | | 800 | 400 | | 800 |
| #1: In AggreWell 800, on the bottom of the well, 300 microwells having a shape of an inverted pyramid of $800\mu$m $\times$ $800\mu$m are inscribed in each well. <br> #2: In AggreWell 400, on the bottom of the well, 1,200 microwells having a shape of an inverted pyramid of $400\mu$m $\times$ $400\mu$m are inscribed in each well. | | | | | | |

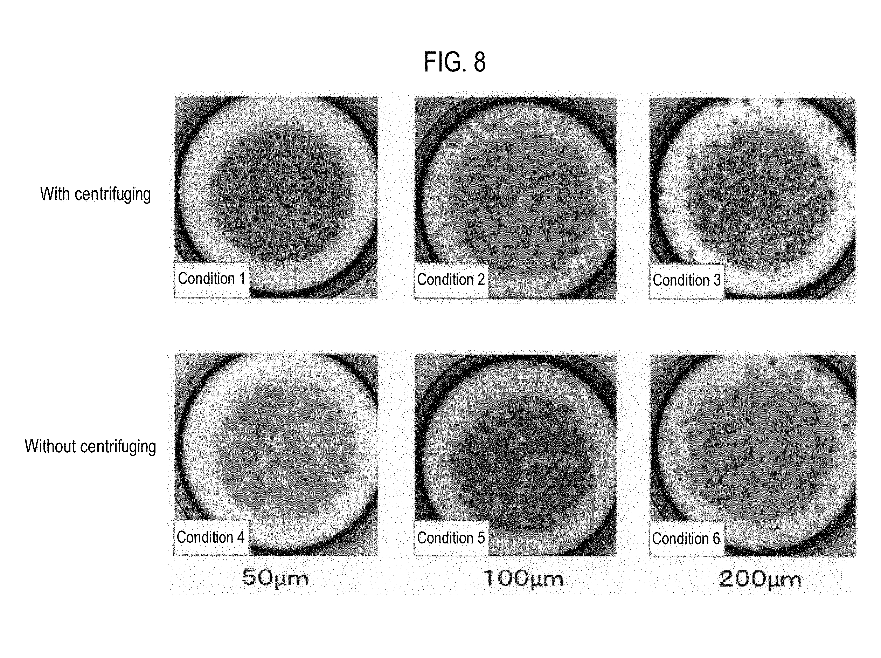

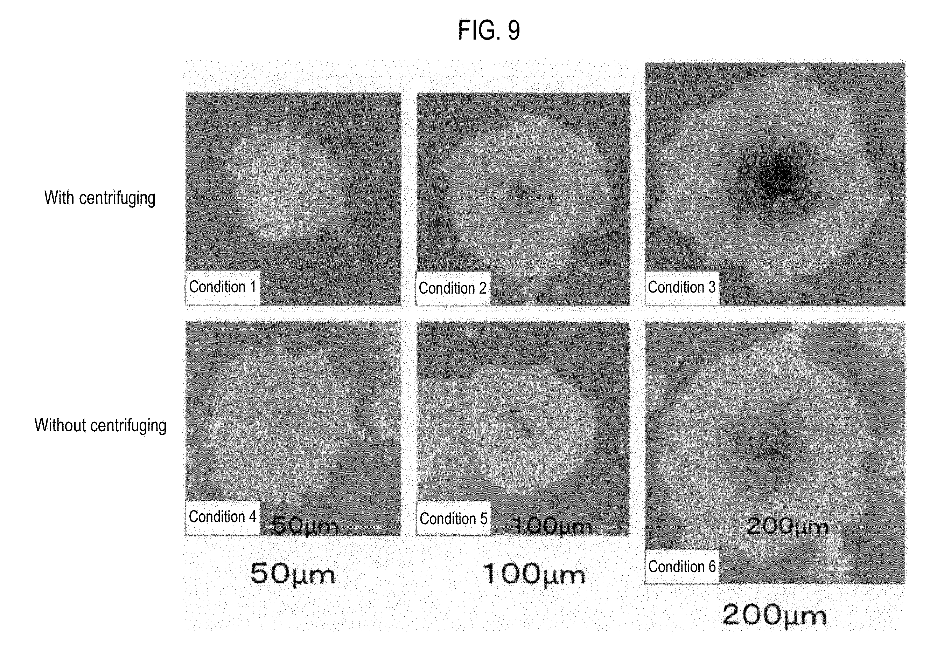

Cells were placed in the wells of AggreWell under the conditions as listed in Table 1. Centrifugation may be conducted at 2000 g for 5 min under any one of conditions 1 to 3. These AggreWells were statically incubated for 24 hrs to form cell aggregates, and the cell aggregates were seeded without breaking them up in 6-well plates. When the plates became confluent or the colony sizes reached 1 mm, the same procedures for passaging as described above were repeated under the conditions as listed in Table 1, and then the colonies obtained at 7 days after being seeded in the 5th passage were observed using a phase contrast microscope. Consequently, the results showed that although passaging under any condition was successful (**FIG. 8**), the colony number under condition 1 was clearly low (**FIG. 8**-upper left). Also, as a result of observation during the procedure of passaging, the cells in colonies of cell aggregates having large sizes (100 μm and 200 μm) seemed to be easily multilayered at 7 days after being seeded (**FIG. 9**). Further, the colonies were verified in the 3rd passage for Test example 4 and in the 4th passage for the others.

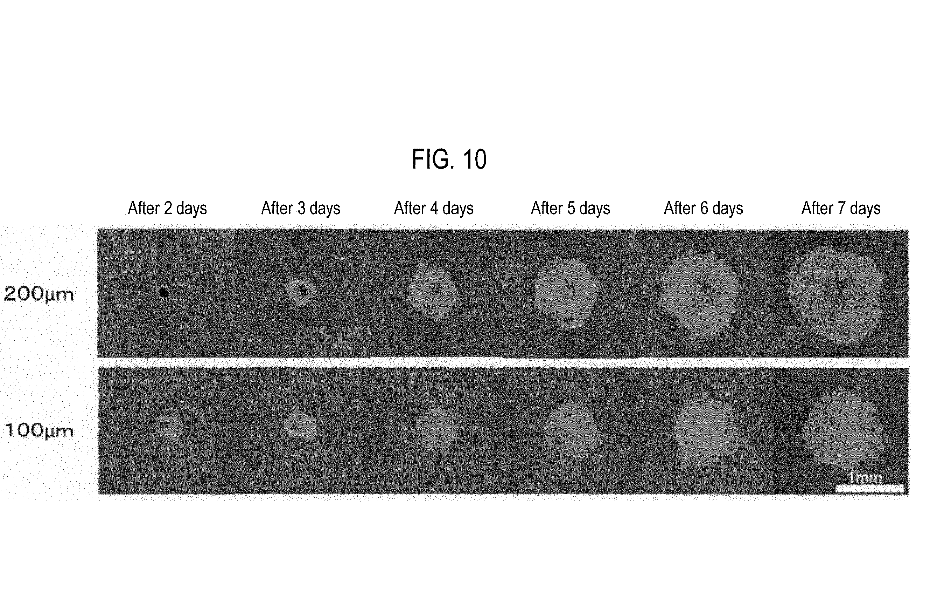

The spreading statuses of the colonies obtained at 2 days to 7 days after being seeded in the 5th passage were observed in a time-lapse manner. As a result, under condition 5, cell aggregates rapidly spread to form colonies, but under condition 6, cell aggregates did not completely spread and grew to diameters in which the cells are subject to be passaged with the multilayered parts remaining (**FIG. 10**).

In the conventional culture of pluripotent stem cells, cell masses have been passaged in an almost monolayered state. However, in the method of this embodiment, multilayered cell aggregates were formed using AggreWell and then seeded. In accordance with the embodiments in Examples 1 to 3, these cell aggregates spread and were monolayered under many conditions during the culture thereof.

The survival rates of colonies after being passaged under each condition were investigated. The survival rates (%) were calculated using the following equation:

<Equation 1>

$$\text{Survival rate (\%)} = (\text{number of survival colonies} / \text{number of seeded cell aggregates}) \times 100$$

by dividing the counted number of colonies on the dish at 3 days after being seeded by the number of seeded cell aggregates.

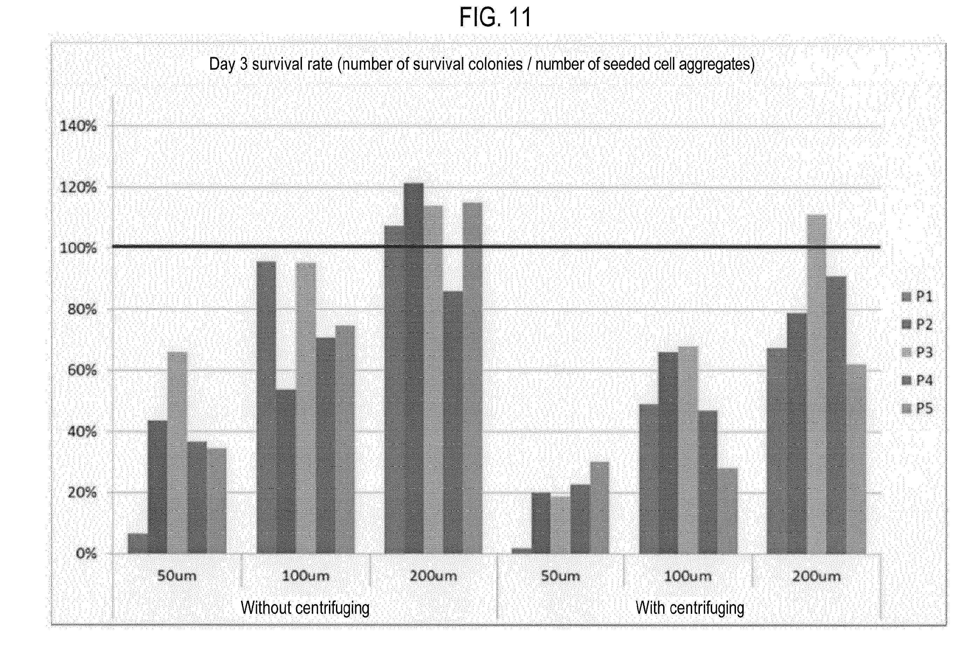

Consequently, the survival rates appeared to be improved under conditions of large cell aggregates (**FIG. 11**). Also, the survival rates appeared to be enhanced under conditions with no centrifuging (**FIG. 11**).

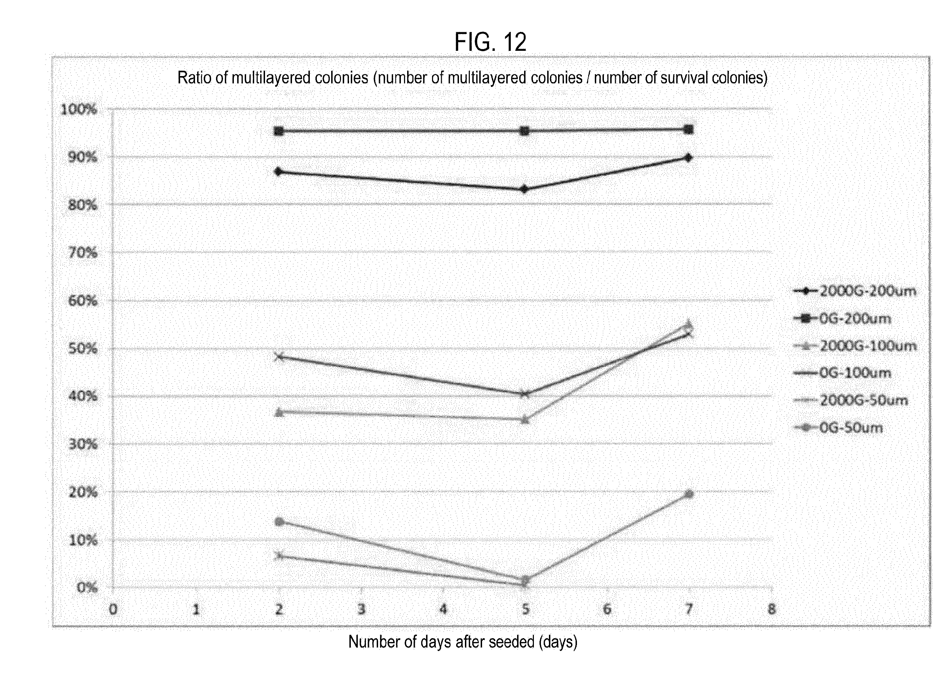

Next, the ratios of colonies containing multilayered cells under each condition were compared. As a result, under conditions 3 and 6 of large cell aggregates, the ratios of multilayered colonies increased, under conditions 1 and 4 of small cell aggregates, those were low (**FIG. 12**). Also, the multilayered colonies were counted as multilayered colonies by distinguishing colonies which were multilayered more than half of the colony area with the naked eye. And the same count procedure as described above was repeated after the 4th passage.

As a result of comparing the growth curves of cells passaged under each conditions, there were no significant differences between the growth curves under any conditions (data not shown).

Further, the recovery rates (%) of colonies after being passaged under each condition were investigated. The survival rates (%) were calculated using the following Equation 2:

<Equation 2>

$$\text{Recovery rate (\%)} = (\text{number of recovered cells} / \text{number of seeded cells}) \times 100$$

by obtaining the ratios between the cell numbers. Because the passaging of cells were conducted until the colony sizes exceeded 1 mm in diameter or until the dishes became confluent, the culture days between passaging appeared to vary depending on the condition (Table 2).

<Table 2> culture days between passages (days)

| Passage number | Without centrifugation | | | With centrifugation | | |
|---|---|---|---|---|---|---|
| | 50 μm | 100 μm | 200 μm | 50 μm | 100 μm | 200 μm |
| 1st | 13 | 6 | 5 | 13 | 7 | 7 |
| 2nd | 7 | 6 | 7 | 9 | 7 | 7 |
| 3rd | 7 | 5 | 7 | 10 | 4 | 4 |
| 4th | 7 | 7 | 7 | 6 | 7 | 7 |
| 5th | 6 | 6 | 6 | 12 | 8 | 8 |

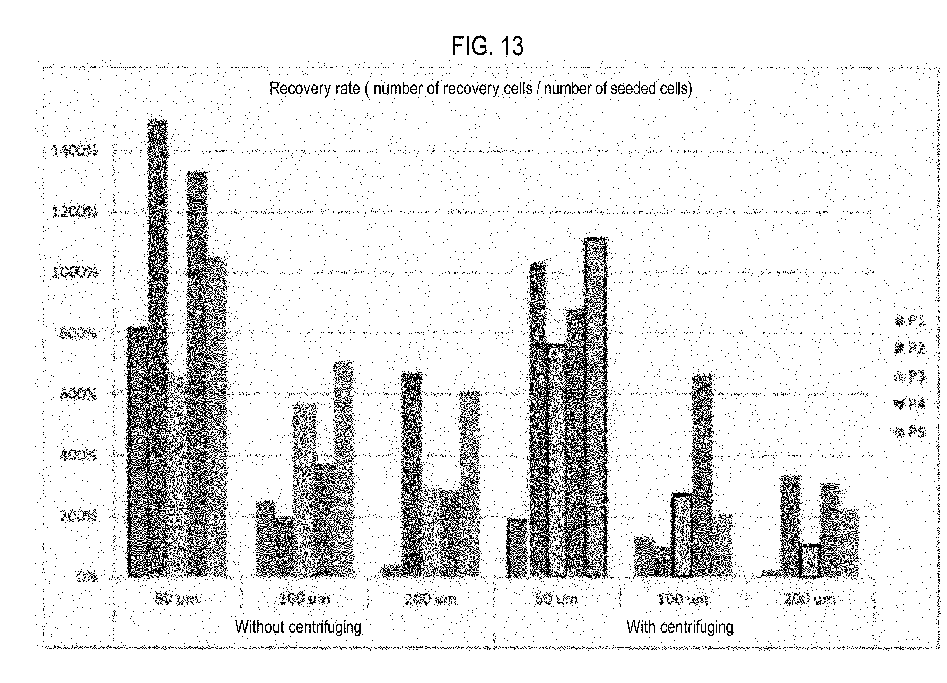

However, the results under all conditions showed recovery rates equivalent to or more than those in the conventional dish cultures (showing 300 to 400%) (**FIG. 13**).

Further, the cell death rates (%) after being passaged under each condition were compared. Cell counting was carried out at 2 days after being seeded. The cell death rates (%) were calculated using the following equation:

<Equation 3>

Cell death rate (%) = [(number of dead cells due to the inability to adhere + number of dead cells during proliferation) / number of seeded cells] × 100.

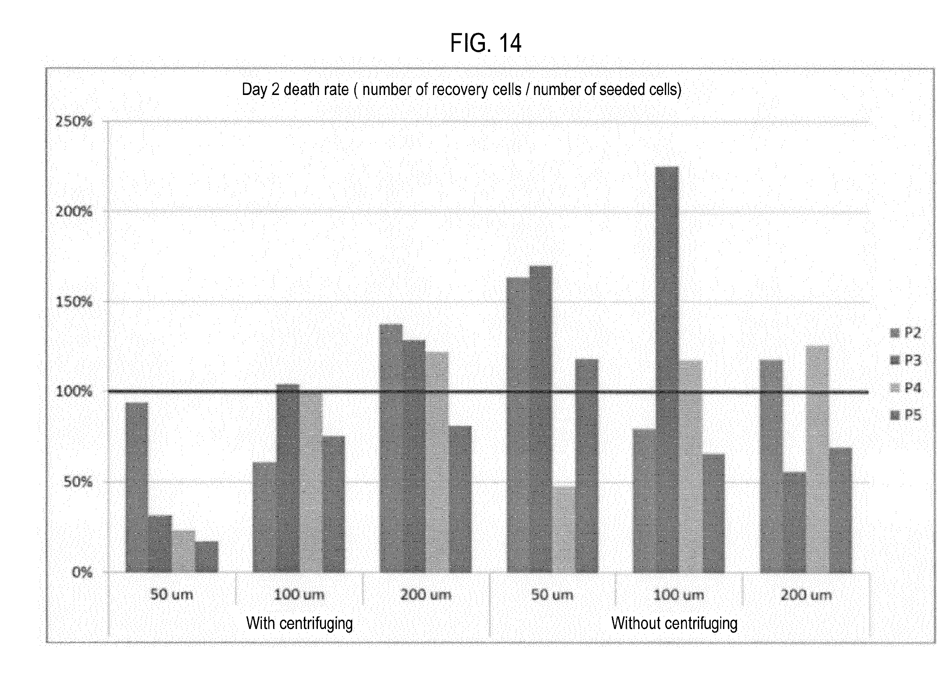

by obtaining the ratios between the cell numbers. Consequently, there were no obvious differences between the cell death rates according to the conditions, though the cell death rate under condition 1 appeared to be a little low (**FIG. 14**). Also, the values of cell death rates were more than 100%, which are considered to be a result of cell proliferation.

As such, pluripotent stem cells could be favorably cultured under various conditions, but the culture results thereafter varied according to the differences in the sizes of cell aggregates or the conditions for forming cell aggregates. In other words, these results clearly show that when cell aggregates are large, they spread slowly and do not complete spread until the following passage; in the case cell aggregates are formed by centrifugation, they become firm such that they are difficult to break down but they spread slowly; when cell aggregates are small, the recovery rate of cells improves but the growth of colonies takes time; and in the case cell aggregates are formed in a shortened time with no centrifuging, they become soft such that they easily break down but spread rapidly.

Furthermore, in the case of conducting passaging under any one condition selected from the above conditions, the sizes of the colonies growing were relatively even (**FIG. 8**). These results suggest that the method of the present invention is superior in terms of cell product quality and culture efficiency.

Example 3-2: Study of proper size of cell aggregates

In Example 3-1, it could be found that the sizes of the formed cell aggregates affected the subsequent cultures. In this embodiment, the proper sizes of cell aggregates were examined in more detail.

First, a cell suspension was obtained according to the same method as described in Example 1. Then, the cell concentration in the obtained cell suspension was appropriately adjusted to form cell aggregates, and the correlation between the diameter of the cell aggregate (y) and the number of contained cells per cell aggregate (x) was investigated.

Cells were statically incubated in wells for 24 hrs to form cell aggregates, the cell aggregates seeded on the culture side of a culture vessel, and the diameters of the cell aggregates were immediately measured thereafter. The measurement was conducted using an optical microscope and the diameters of the cell aggregates were calculated by obtaining areas of the cell aggregates computed from their optical microscopic images on the assumption that the cell aggregates were spherical.

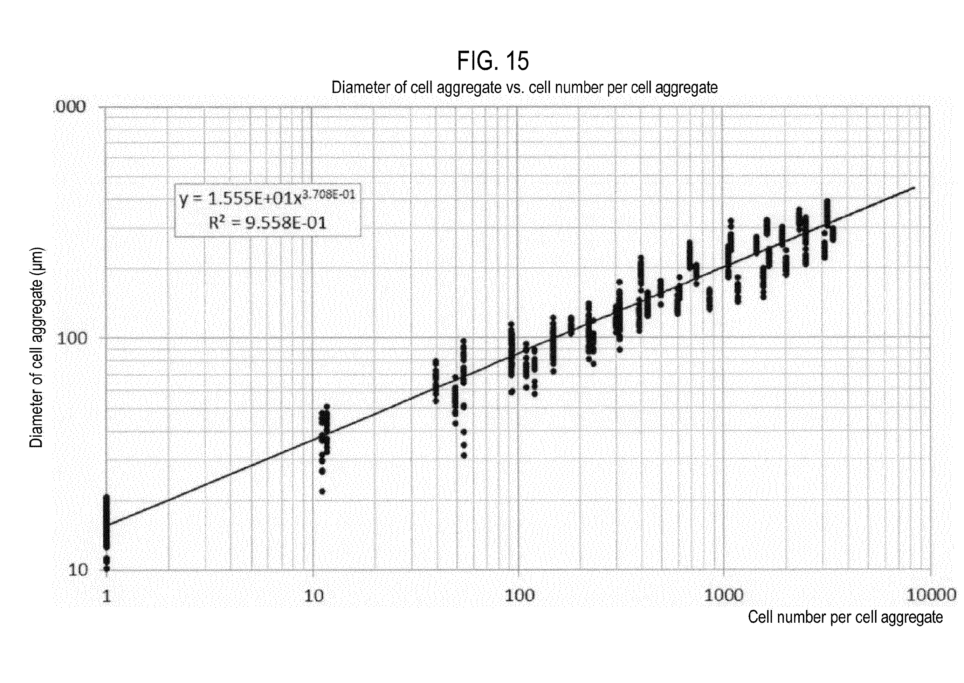

As a result, it was found that the diameter of the cell aggregate (y) has a correlation ($R^2$=0.96) with the number of contained cells per cell aggregate (x) as expressed in the following equation (**FIG. 15**).

<Equation 4>

$$y = 1.56 \times 10 x^{0.371}$$

In this embodiment of the present invention, the cell concentrations were adjusted in order to allow an average cell number per cell aggregate to range from 11 to 3,415 cells (**FIG. 15**), and cell aggregates could be favorably formed under all examined conditions.

In Example 3-1, when the cell aggregates were large, they slowly spread on the culture side, for example, it could be found that the cell aggregates did not completely spread until 8 days after being seeded because of their large size. Therefore, the correlation between the cell aggregate spreading after being seeded and the cell aggregate size was investigated in detail.

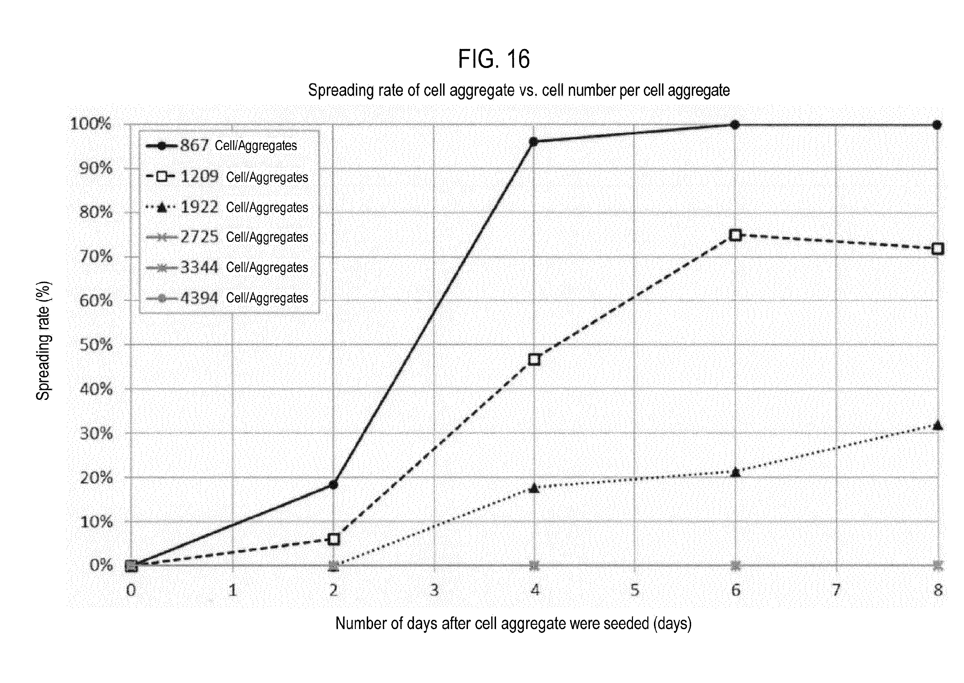

As a result, in the case of cell aggregates having an average cell number per formed cell aggregate of 867 cells (corresponding to 195 μm in diameter), it was observed that almost 100% of the cell aggregates spread at 6 to 8 days after being seeded (**FIG. 16**). Meanwhile, in the case of cell aggregates having an average cell number per formed cell aggregate of 1,209 cells (corresponding to 216 μm in diameter) or more, the cell aggregates did not complete spreading (**FIG. 16**). In this regard, in terms of the spread property, it became clear that the average cell number per cell aggregate may be preferably less than or equal to 1,209 cells (corresponding to 216 $\mu$m in diameter), more preferably 867 cells (corresponding to 195 $\mu$m in diameter).

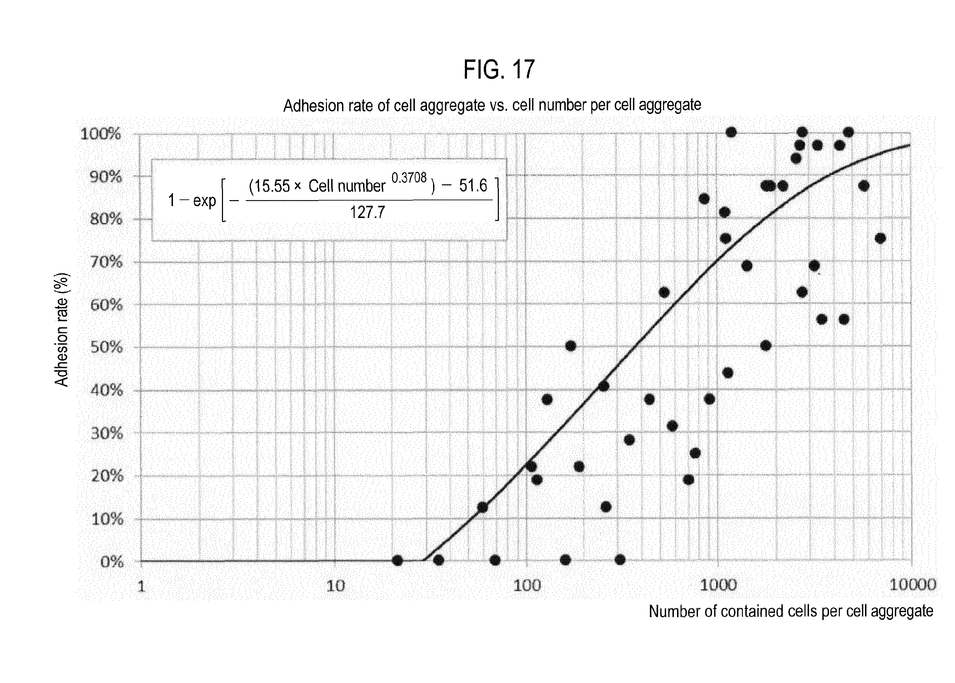

Further, in terms of improving the cell proliferation rates, in order to obtain a guideline for optimizing the sizes of the cell aggregates, after the cell aggregates were seeded on the culture side, it was investigated in detail the correlation between the cell adhesion rate to the culture side and the size of the cell aggregate. Consequently, it was clarified that the cell adhesion rate to the culture side increased as the number of contained cells per cell aggregate increased (as the size of the cell aggregates became bigger) (**FIG. 17**). When a regression analysis was conducted for the results described above, it was found that the adhesion rate of cell aggregate to the culture side (y) has a correlation with the number of contained cells per cell aggregate (x) as illustrated in the following equation (**FIG. 17**).

<Equation 5>

$$y = 1 - e^{\left[\frac{\left(15.55x^{0.37078}\right) - 51.6}{127.7}\right]}$$

In other words, it became clear that the adhesion rate of cell aggregate became lowered when the average cell number per cell aggregate is less than or equal to about 28 cells (corresponding to about 51.6 $\mu$m in diameter). So, in terms of adhesion property, in theory, the average cell number per cell aggregate may be preferably adjusted to be more than about 28 cells (corresponding to about 51.6 $\mu$m in diameter). However, because the variations in survival rates are so large, especially where the survival rates are low (e.g., average number of cells around 28 cells (i.e., corresponding to around 50 $\mu$m in diameter)), it can be thought that the approximation error thereof became relatively large, and thus, even though cell aggregates have an average cell number less than or equal to 28 cells (i.e., 50 $\mu$m), it does not mean that their culture cannot be conducted.

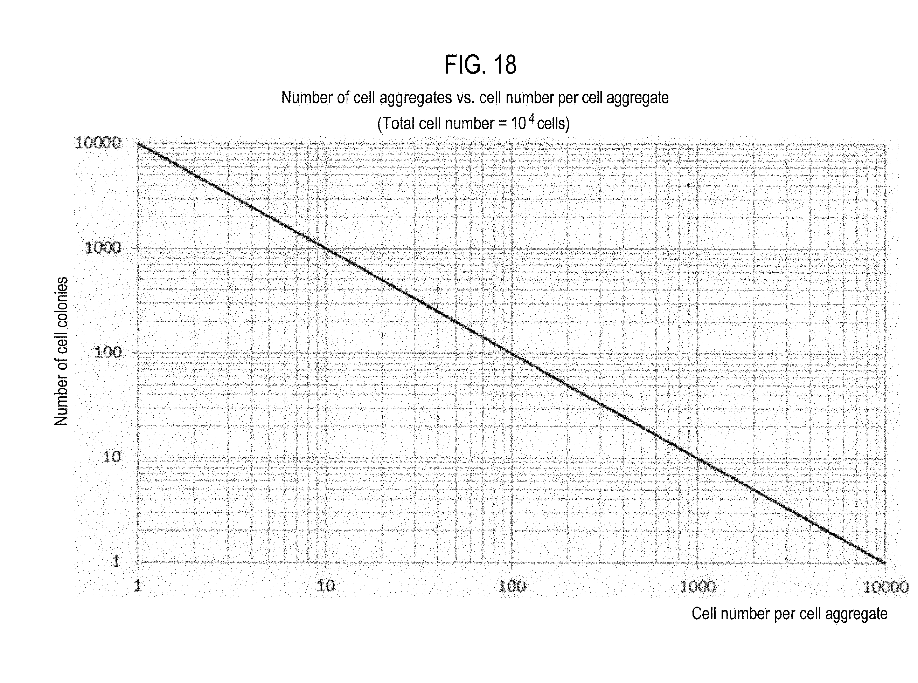

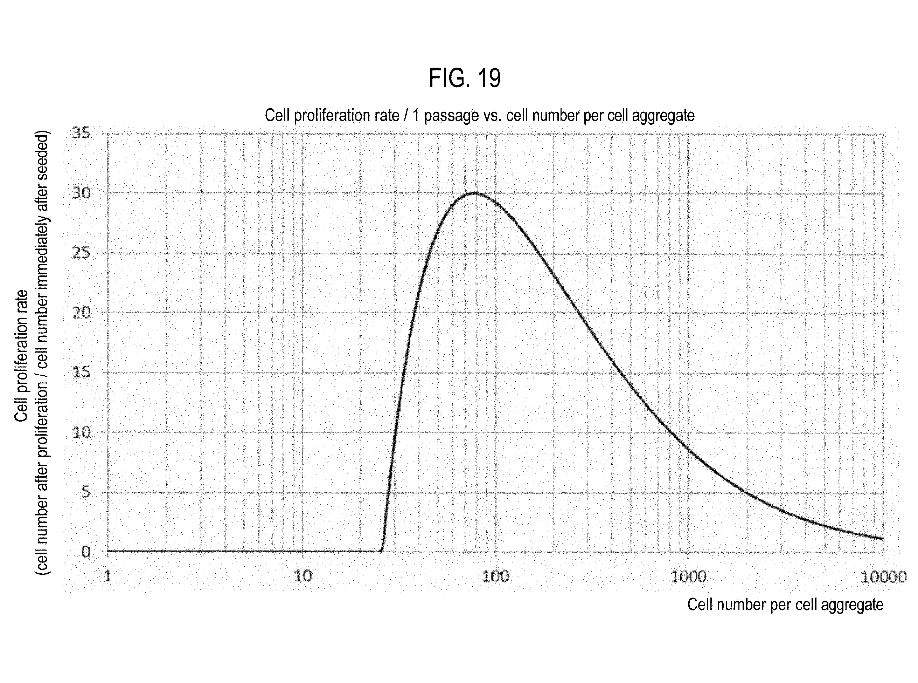

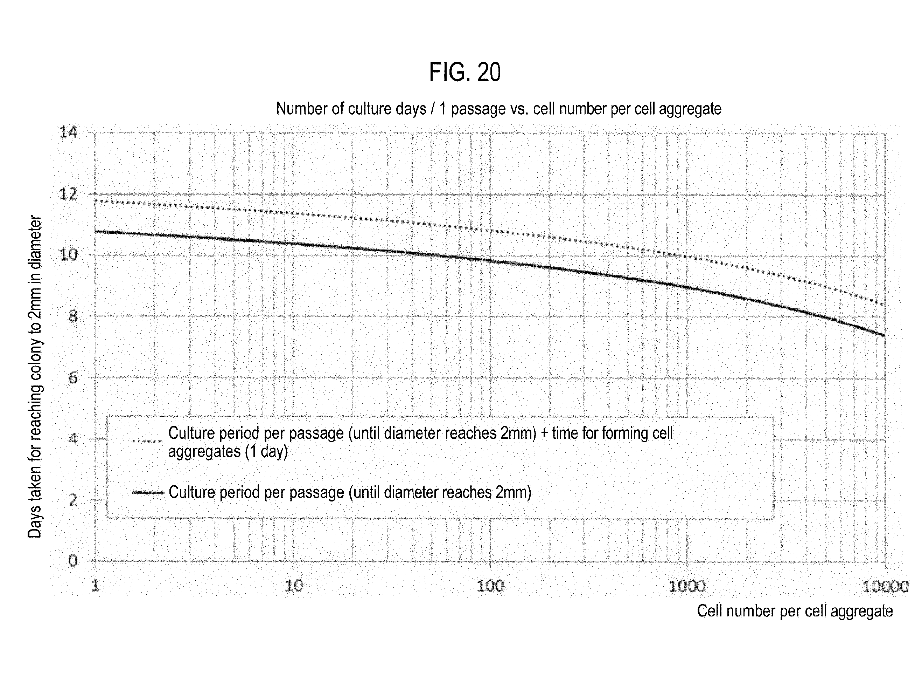

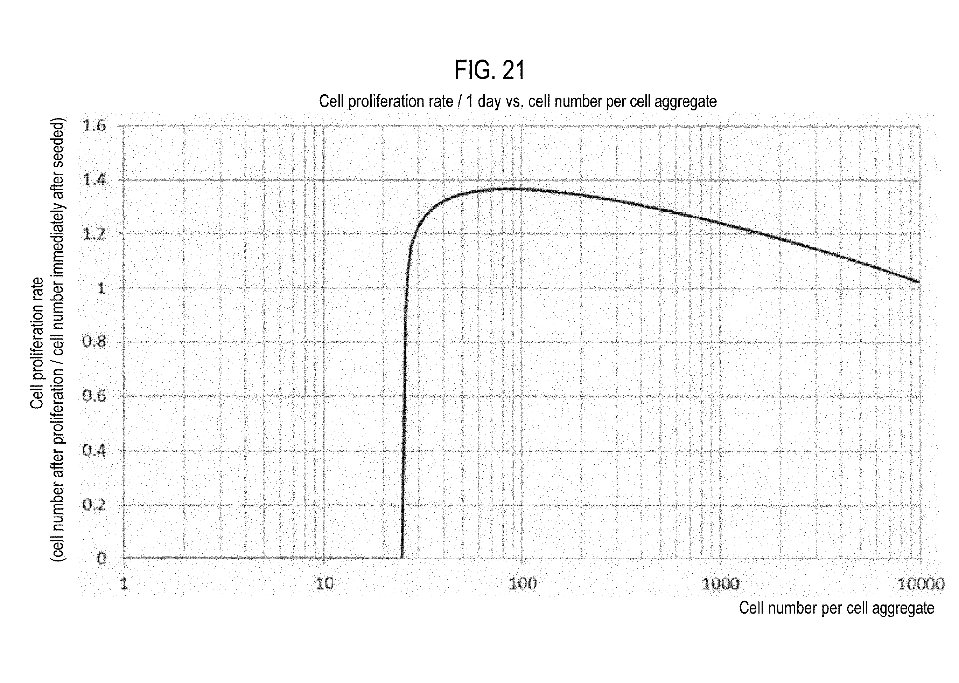

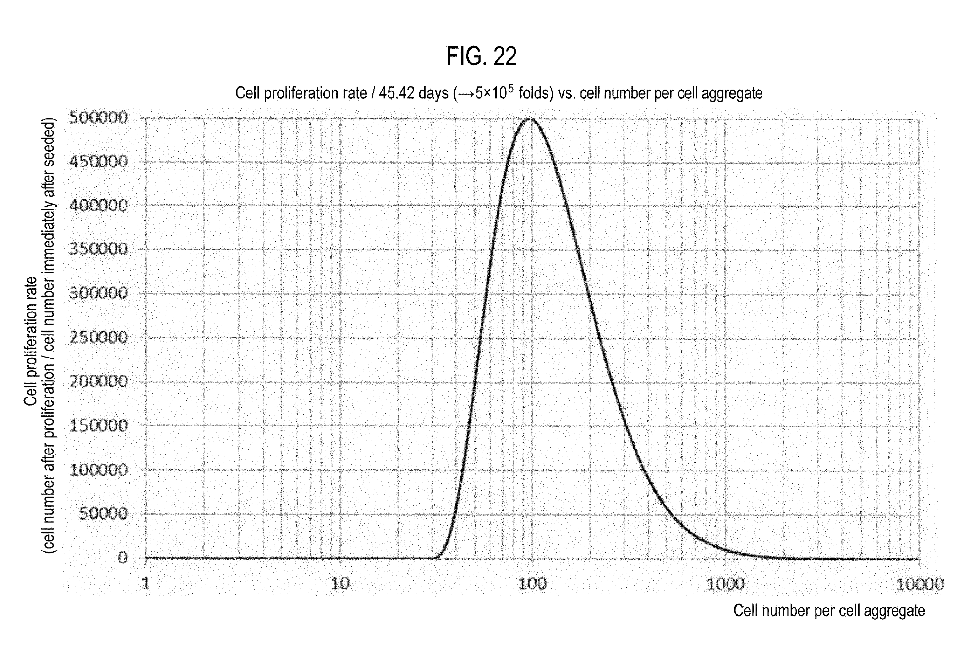

The present inventors also examined the optimum size of cell aggregate in terms of cell proliferation rate. First, in the case cell numbers used in passage were equal, the correlation between the number of contained cells per cell aggregate and the number of obtained cell aggregates are shown in **FIG. 18.** In addition, from these results of adhesion rate of obtained cell aggregates (**FIG. 17**) and **FIG. 18,** the cell proliferation rates until the subsequent passage (cell proliferation rates per passage) were calculated. Specifically, passage timing was when the sizes of colonies formed through spreading of the cell aggregates reached 2 mm in diameter, and the cell numbers per unit area in colonies were 4,000 cells/mm$^2$. In other words, passaging was conducted at the time of reaching the cell number per colony of 12,566 cells. Also, the cell proliferation rates after being passaged until the subsequent passage were calculated considering the correlation between the size of cell aggregate, number of contained cells per cell aggregate and adhesion rates of cell aggregates (**FIG. 17** and **FIG. 18**). As a result, a bulging shaped graph was obtained showing that the cell proliferation rate became maximized when the number of contained cells per cell aggregate was 76 cells (**FIG. 19**). Further, as a result of analyzing the sizes of cell aggregates and days until the following passage, it was found that there was a correlation shown in **FIG. 20.** Accordingly, the cell proliferation rates per day were also calculated. As a result, a bulging shaped graph was obtained showing that the cell proliferation rate was maximized when the number of contained cells per cell aggregate was 96 cells (**FIG. 21**). Besides, the days taken until the cells became $5 \times 10^5$ fold were calculated under the condition that cell proliferation rate became maximized as shown in **FIG. 21,** and thus it was found that theoretically 45.42 days were necessary therefor. Accordingly, the correlation between the size of the cell aggregate and cell proliferation rate after 45.42 days was investigated (**FIG. 22**). As a result, in the case the number of contained cells per cell aggregate was 42 to 496 cells, proliferation rates more than or equal to 1/10 of the maximum cell proliferation rate could be expected. Also, in the case the number of contained cells per cell aggregate was 55 to 217 cells, proliferation rates more than or equal to 1/2 of the maximum cell proliferation rate could be expected.

In this regard, it is clear that cell culture efficiency can be remarkably improved by controlling the sizes of formed cell aggregates, and the guideline for optimizing the sizes of cell aggregates has been established.

In general culture methods, the sizes of cell masses when being seeded are non-uniform. While, in the method of the present invention, the sizes of cell aggregates can be easily controlled to be uniform by controlling the cell numbers seeded in each microwell, and each sizes of the cell aggregates thus obtained can be regularly aligned. Therefore, in the method of the present invention, the proliferation efficiency of pluripotent stem cells can be easily improved.

Example 4: Study of the method of seeding cell aggregates on the culture side of a culture vessel

In the Examples described above, the cell aggregates formed in microwells were harvested by pipetting and passaged to a new culture vessel, but it was necessary to take time for the pipetting work and be careful in order not to break down the cell aggregates. Therefore, in this embodiment of the present invention, more simple methods of seeding cell aggregates were examined.

The present inventors examined a method using a closed culture vessel in which a side having microwells and a culture side were equipped and both sides are placed opposite to each other. In the side having microwells of the closed culture vessel used, microwells in the shape of a chamfer plane bottom having an upper opening in the shape of a 1,000 $\mu$m $\times$ 1,000 $\mu$m square were aligned in the form of a checkerboard. Also, the culture side was coated by BD Matrigel (trademark).

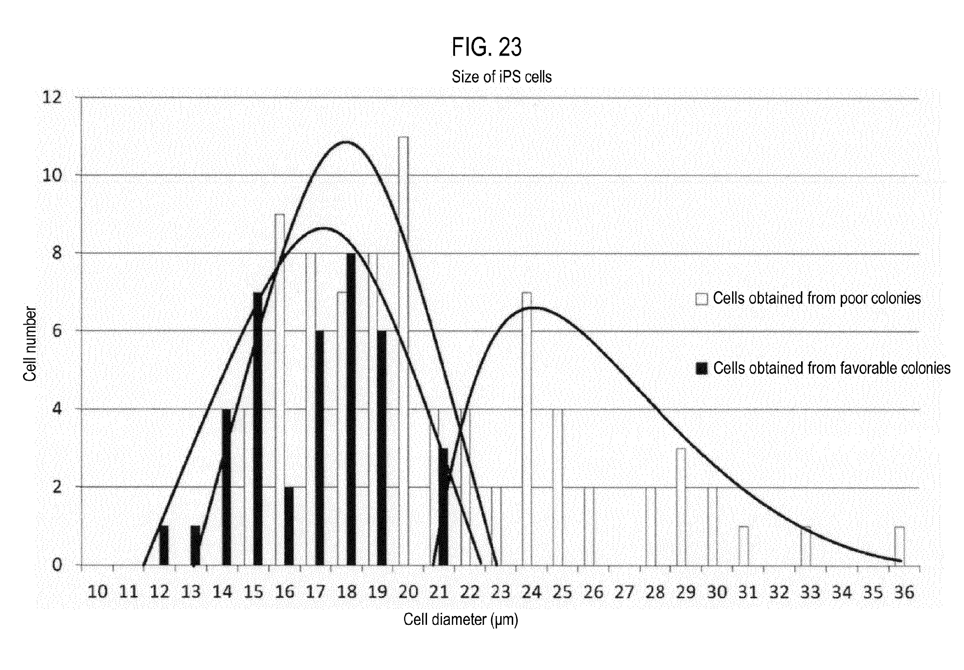

Then, the method described in Example 1 was carried out to obtain a cell suspension of iPS cells dispersed into single cells. The obtained cell suspension was injected in the closed culture vessel described above, the microwells-side down, and statically incubated at 37 degrees C for 24 hrs under 5% $CO_2$ atmosphere. After observing the formation of cell aggregates using an optical microscope, the closed culture vessel was inverted the culture side down, which allowed the cell aggregates to vertically drop from the microwells onto the culture side. **FIG. 23** is a view illustrating the arrangements of the cell aggregates dropped onto the culture side. As shown in **FIG. 23,** the cell aggregates were regularly aligned on the culture side. Such arrangement of cell aggregates reflects the arrangement pattern of the used microwells, the gaps between the cell aggregates in **FIG. 23** corresponded with the pitches (1,000 $\mu$m) of the microwells.

In this regard, it is clear that the cell aggregates in microwells can be dropped onto the culture side of a culture vessel while maintaining the arrangement pattern of the microwells. It is thought that the seeded positions of the cell aggregates can be freely controlled by changing the arrangements of the microwells. Further, it is clarified that the processes of seeding and controlling the seeded positions can be conducted by the very easy task of inverting a vessel.

In accordance with Examples 1 to 3, it was possible to favorably culture pluripotent stem cells dispersed into single cells while maintaining undifferentiated state by forming cell aggregates immediately after dispersion. Also, cell aggregates of uniform sizes could be simply formed by seeding the cell suspension in a culture vessel having a side in which multiple microwells were arranged. Further, cell aggregates rapidly spread after being seeded and then cells proliferated favorably. Due to the uniform sizes of the formed cell aggregates, the spreading speed and proliferation speed thereafter of cell aggregates were also uniform. In addition, because of the uniformly sized cell aggregates, the efficiency of subculturing was improved and the management for cell product quality was facilitated. Furthermore, in accordance with Example 4, cell aggregates in microwells could be seeded on the culture side of a culture vessel through simple manipulation. The arrangement of seeded cell aggregates reflected the arrangement pattern of the microwells, and it was found that it was possible to precisely seed cell aggregates by simple manipulation. As such, according to the method of the present invention, the formation of uniform cell aggregates or uniform seeding of cell aggregates can be carried out by very simple mechanical manipulation. The method of the present invention can facilitate the maintenance of the product quality of pluripotent stem cells and pioneers a way of totally automating the subculturing of pluripotent stem cells.

Example 5: Sorting of cells based on sizes

In accordance with the Examples described above, it can be known that pluripotent stem cells, even in the case of being dissociated into single cells, can still be cultured well thereafter by rapidly forming cell aggregates. In this embodiment, using such an advantage of the present invention of being capable of dispersing into single cells, the sorting possibility of cells based on sizes was evaluated.

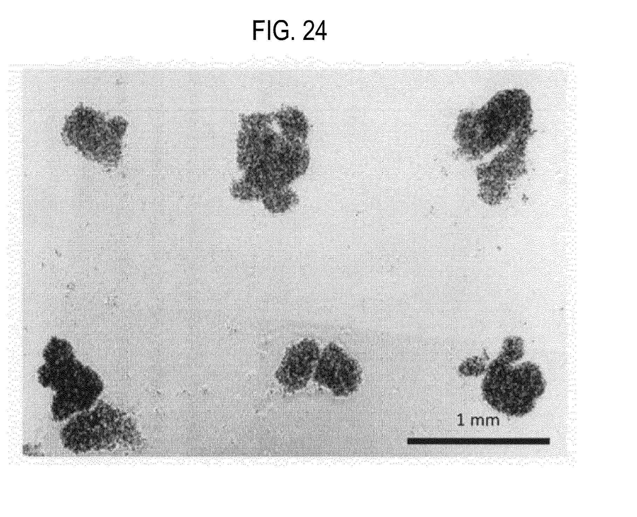

With the naked eye, iPS cell colonies determined as undifferentiated (i.e., "favorable") and iPS cell colonies determined as partially differentiated (i.e., "poor") were each isolated by pipetting, continuously dispersed using enzymes, and then subjected to microscopic observation to analyze the size distribution of single cells. As a result, it could be verified that cells from the colonies determined as "favorable" appeared to each have uniform sizes ranging from 14 to 20 $\mu$m centering around 17 $\mu$m, while among cells from the colonies determined as "poor", cells having sizes more than or equal to 22 $\mu$m or more than or equal to 23 $\mu$m could be observed (**FIG. 24**). The cells having sizes more than or equal to 22 $\mu$m or more than or equal to 23 $\mu$m were expected to be cells which initiated differentiation in colonies as compared with cells from the colonies determined as "favorable", and from the analysis of phase contrast microscopy, evaluated as cells differentiated and losing pluripotency. These results indicate that it is possible to remove cells which initiate differentiation to lose pluripotency (differentiated cells) by sorting manipulation. Further, the procedure of removing differentiated cells can be carried out by employing a flow cytometry method.

The management of the product quality of pluripotent stem cells is an important issue in the subculturing of pluripotent stem cells. In the present invention, differentiated cells among pluripotent stem cells dispersed into single cells can be removed by simple mechanical manipulation. Therefore, in a sense, the present invention can be said to have pioneered the way to automation of removing differentiated cells in subculture.

**Claims**

1. A method of subculturing pluripotent stem cells, comprising the steps of:

    (a) dispersing cell masses of pluripotent stem cells during passaging;
    (b) seeding the dispersed cells in microwells;
    (c) forming cell aggregates from the seeded cells in the microwells; and
    (d) seeding the formed cell aggregates on a culture side of a culture vessel.

2. The method of Claim 1, wherein step (d) comprises the step of (d') inverting a vessel equipped with microwells to drop cell aggregates to a culture side of a culture vessel.

3. The method of Claim 1 or 2, wherein in step (a), the cell masses are dissociated into cell masses each containing 1 to 100 cells.

4. The method of Claim 3, wherein in step (a), the cell masses are dissociated into cell masses each containing 1 to 10 cells.

5. The method of Claim 4, wherein in step (a), the cell masses are dissociated into single cells.

6. The method of any one of Claims 1 to 5, wherein in step (b), an average cell number of cells seeded in each microwell is 10 to 3,500 cells per well.

7. The method of Claim 6, wherein in step (b), an average cell number of cells seeded in each microwell is 25 to 870 cells per well.

8. The method of Claim 7, wherein in step (b), an average cell number of cells seeded in each microwell is 40 to 500 cells per well.

9. The method of Claim 8, wherein in step (b), an average cell number of cells seeded in each microwell is 55 to 220 cells per well.

10. The method of any one of Claims 1 to 9, wherein step (c) comprises the step of statically incubating the cells in the microwells for a sufficient time to form cell aggregates.

11. The method of Claim 10, wherein the step (c) comprises the step of statically incubating the cells in the microwells for 8 to 24 hours.

12. The method of Claim 11, wherein the step (c) comprises the step of statically incubating the cells in the microwells for 8 to 12 hours.

13. The method of any one of Claims 1 to 12, wherein step (c) is carried out without centrifuging.

14. The method of any one of Claims 1 to 13, wherein the pluripotent stem cells are human pluripotent stem cells.

15. The method of Claim 14, wherein the human pluripotent stem cells are human ES cells or human iPS cells.

16. The method of any one of Claims 1 to 15, which further comprises the step of (a') removing differentiated cells after step (a).

17. The method of Claim 16, wherein the step of (a') removing differentiated cells comprises the step of removing differentiated cells by sorting.

18. The method of Claim 17, wherein cells having a diameter more than a threshold value (the threshold value is more than or equal to 20 $\mu$m) are removed by sorting.

19. The method of Claim 18, wherein cells having a diameter more than a threshold value (the threshold value is more than or equal to 23 $\mu$m) are removed by sorting.

20. The method of Claim 16, wherein the step of (a') removing differentiated cells is conducted based on whether a cell surface marker is expressed or not.

21. The method of Claim 20, wherein the cell surface marker is an undifferentiation marker expressed on surface of pluripotent stem cells.

22. The method of Claim 21, wherein the undifferentiation marker is one or more undifferentiation markers selected from the group consisting of alkali phosphatase, SSEA-3, SSEA-4, TRA-1-60 and TRA-1-81.

23. A closed culture vessel in which a side equipped with microwells and a culture side are equipped and both sides are placed opposite to each other.

24. The closed culture vessel of Claim 23, wherein the microwells each have a shape in which inner circumference becomes smaller toward the bottom.

25. The closed culture vessel of Claim 24, wherein the microwells each have a rounded bottom, a V bottom, a U bottom or a chamfer plane bottom.

26. The closed culture vessel of any one of Claims 23 to 25, which is equipped with arrayed multiple microwells.

27. The method of Claim 2, wherein step (d') is conducted by inverting the closed culture vessel of any one of Claims 23 to 26.

28. A totally automated subculture system of pluripotent stem cells for accomplishing the method of any one of Claims 1 to 22 and 27.

FIG. 1

FIG. 2

FIG. 3

## FIG. 4

Phase contrast image | DAPI | Nanog | Oct3/4

With centrifuging

Phase contrast image | DAPI | Nanog | Oct3/4

without centrifuging

# FIG. 5

| 0 hr | 1 hr | 2 hrs | 3 hrs | 4 hrs | 5 hrs |
|------|------|-------|-------|-------|-------|

| 6 hrs | 7 hrs | 8 hrs | 9 hrs | 10 hrs | 11 hrs |
|-------|-------|-------|-------|--------|--------|

23 hrs

# FIG. 6

8 hrs                    10 hrs

12 hrs                   24 hrs
                    (comparative sample)

|  | 8 hrs | 10 hrs | 12 hrs | 24 hrs |
|---|---|---|---|---|
| Number of colonies having a diameter of 1mm or larger | 84 | 36 | 19 | 0 |

# FIG. 7

8 hrs                    10 hrs

12 hrs                    24 hrs
(comparative sample)

| | 8 hrs | 10 hrs | 12 hrs | 24 hrs |
|---|---|---|---|---|
| Number of colonies having a diameter of 1mm or less | 234 | 199 | 182 | 190 |
| Number of cells | $1.44 \times 10^6$ | $1.12 \times 10^6$ | $9.8 \times 10^5$ | $1.19 \times 10^5$ |

FIG. 8

With centrifuging

Condition 1  Condition 2  Condition 3

Without centrifuging

Condition 4  Condition 5  Condition 6

50µm  100µm  200µm

EP 2 949 746 A1

# FIG. 9

With centrifuging

Condition 1

Condition 2

Condition 3

Without centrifuging

Condition 4    50µm

Condition 5    100µm

200µm

Condition 6

50µm

100µm

200µm

# FIG. 10

|  | After 2 days | After 3 days | After 4 days | After 5 days | After 6 days | After 7 days |
|---|---|---|---|---|---|---|
| 200μm | | | | | | |
| 100μm | | | | | | 1mm |

FIG. 11

Day 3 survival rate (number of survival colonies / number of seeded cell aggregates)

FIG. 12

Ratio of multilayered colonies (number of multilayered colonies / number of survival colonies)

Number of days after seeded (days)

Legend:
- 2000G-200um
- 0G-200um
- 2000G-100um
- 0G-100um
- 2000G-50um
- 0G-50um

FIG. 13

Recovery rate ( number of recovery cells / number of seeded cells)

FIG. 14

Day 2 death rate ( number of recovery cells / number of seeded cells)

EP 2 949 746 A1

FIG. 15

Diameter of cell aggregate vs. cell number per cell aggregate

$y = 1.555E+01x^{3.708E-01}$

$R^2 = 9.558E-01$

Diameter of cell aggregate (μm)

Cell number per cell aggregate

# FIG. 16

Spreading rate of cell aggregate vs. cell number per cell aggregate

## FIG. 17

Adhesion rate of cell aggregate vs. cell number per cell aggregate

$$1 - \exp\left[ - \frac{(15.55 \times \text{Cell number}^{0.3708}) - 51.6}{127.7} \right]$$

# FIG. 18

Number of cell aggregates vs. cell number per cell aggregate

(Total cell number = $10^4$ cells)

EP 2 949 746 A1

EP 2 949 746 A1

# FIG. 19

Cell proliferation rate / 1 passage vs. cell number per cell aggregate

# FIG. 20

Number of culture days / 1 passage vs. cell number per cell aggregate

Culture period per passage (until diameter reaches 2mm) + time for forming cell aggregates (1 day)

Culture period per passage (until diameter reaches 2mm)

EP 2 949 746 A1

# FIG. 21

Cell proliferation rate / 1 day vs. cell number per cell aggregate

EP 2 949 746 A1

# FIG. 22

Cell proliferation rate / 45.42 days ($\rightarrow 5\times 10^5$ folds) vs. cell number per cell aggregate

FIG. 23

Size of iPS cells

Cells obtained from poor colonies

Cells obtained from favorable colonies

Cell number

Cell diameter (μm)

EP 2 949 746 A1

FIG. 24

## INTERNATIONAL SEARCH REPORT

| International application No. |
|---|
| PCT/JP2014/051362 |

A. CLASSIFICATION OF SUBJECT MATTER
*C12N5/07*(2010.01)i, *C12M1/18*(2006.01)i, *C12N5/071*(2010.01)i

According to International Patent Classification (IPC) or to both national classification and IPC

B. FIELDS SEARCHED

Minimum documentation searched (classification system followed by classification symbols)
C12N5/07, C12M1/18, C12N5/071

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
CAplus/MEDLINE/EMBASE/BIOSIS(STN), WPIDS/WPIX(STN),
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | Kanji YAHIRO et al., "Introduction of stem cell culture, differentiation and assay tools", THE CELL, 20 August 2012 (20.08.2012), 44(9), pages 393 to 397, entire text, particularly, abstract, page 394, left column, line 3 to page 396, left column, line 7, fig. 1, 2, 4 | 1,3-15<br>2-16,20-28<br>17-19,28 |
| X<br>Y<br>A | STOVER, A.E., et al., The Generation of Embryoid Bodies from Feeder-Based or Feeder-Free Human Pluripotent Stem Cell Cultures, Methods in Molecular Biology, 2011, 767, pp.391 -398, entire text, particularly, summary, 3.2, 3.2.2, table 1, fig. 2 to 4 | 1,3-12,14,15<br>2-16,20-28<br>17-19,28 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 April, 2014 (09.04.14) | 22 April, 2014 (22.04.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2014/051362 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y<br>A | Hito ES/iPS Saibo kara Kin'itsu na Embryoid Body o Tsukuru Tokushu Plate, Veritas Corp., Website [online], 25 September 2009 (25.09. 2009), [retrieval date 09 April 2014 (09.04. 2014)], Internet <URL:http://www.veritastk.co. jp/news.php?id=345>, entire text | 1,3-12,14,15<br>2-16,20-28<br>17-19,28 |
| Y<br>A | WO 2007/114351 A1  (Asubio Pharma Co., Ltd.), 11 October 2007 (11.10.2007), entire text; particularly, examples; fig. 5, 6, 8<br>& JP 5074382 B          & US 2010/0297767 A1<br>& EP 1992685 A1          & EP 2537921 A1 | 2-16,20-28<br>17-19,28 |
| Y<br>A | JP 2010-233456 A  (Kitakyushu Foundation for the Advancement of Industry, Science and Technology), 21 October 2010 (21.10.2010), entire text; particularly, example 1; fig. 11 (Family: none) | 2-16,20-28<br>17-19,28 |
| Y<br>A | WO 2011/022507 A1  (THE BOARD OF TRUSTEES OF THE LELAND STANFORD JUNIOR UNIVERSITY), 24 February 2011 (24.02.2011), claims; paragraphs [0024], [0067]; examples<br>& JP 2013-502220 A       & US 2012/0220030 A1<br>& EP 2467469 A          & CA 2771303 A<br>& MX 2012002117 A       & IL 218111 D | 16,20-22,28<br>17-19,28 |
| Y<br>A | WO 2011/068879 A2  (RESEARCH DEVELOPMENT FOUNDATION), 09 June 2011 (09.06.2011), page 20, line 9 to page 22, line 7; page 31, line 28 to page 33, line 20; examples<br>& JP 2013-512671 A       & US 2011/0136681 A1<br>& EP 2507363 A          & CA 2782577 A<br>& AU 2010326106 A       & CN 102741397 A<br>& KR 10-2012-0093407 A | 16,20-22,28<br>17-19,28 |
| Y<br>A | WO 2012/087965 A2  (FATE THERAPAUETICS, INC.), 28 June 2012 (28.06.2012), entire text; particularly, claims; examples<br>& AU 2011349446 A       & CA 2822638 A<br>& JP 2014-501108 A | 16,20-22,28<br>17-19,28 |
| Y<br>A | WO 2011/161962 A1  (Kawasaki Heavy Industries, Ltd.), 29 December 2011 (29.12.2011), claims; drawings<br>& US 2013/0130228 A1    & EP 2586872 A1 | 28<br>17-19,28 |

Form PCT/ISA/210 (continuation of second sheet) (July 2009)

**INTERNATIONAL SEARCH REPORT**

| International application No. |
|---|
| PCT/JP2014/051362 |

C (Continuation).   DOCUMENTS CONSIDERED TO BE RELEVANT

| Category* | Citation of document, with indication, where appropriate, of the relevant passages | Relevant to claim No. |
|---|---|---|
| A | Hiromich YOSHIOKA et al., "iPS Saibo Yurai Bunka Saibo to Mibunka iPS Saibo no Isosa ni yoru Hi Shinshuteki Shikibetsu", Dai 64 Kai Abstracts of the Annual Meeting of the Society for Biotechnology, Japan, The Society for Biotechnology, Japan, 25 September 2012 (25.09. 2012), page 112(Yoshi Bango 3Ba10), entire text | 17-19,28 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013010161 A

- JP 2002000178 A


**Non-patent literature cited in the description**

- **WATANABE, K.** A ROCK inhibitor permits survival of dissociated human embryonic stem cells. *Nature bBotechnology,* 2007, vol. 25, 681
- **SPELKE D.P.** Methods for embryoid body formation: the microwell approach. *Methods in Molecular Biology,* 2011, vol. 690, 151-162

- **SHIMAZAKI TAKUYA ; OKADA YOHEI ; YOSIJA-KI DHAKAHITO ; OKANO HIDEYUKI.** Protein, Nucleic acid and Enzyme. *Kioritz Publication,* 2006, vol. 51 (13), 1854-1861